# EUROPEAN PATENT SPECIFICATION

(11) **EP 1 790 277 B1**
(45) Date of publication and mention of the grant of the patent: **21.11.2018**
(21) Application number: 05775083.8
(22) Date of filing: 23.08.2005
(51) Int. Cl.: A61B 1/00

(54) **IMAGE DISPLAY DEVICE AND IMAGE DISPLAY PROGRAM**
BILDANZEIGEVORRICHTUNG UND BILDANZEIGEPROGRAMM
DISPOSITIF D'AFFICHAGE D'IMAGE ET PROGRAMME D'AFFICHAGE D'IMAGE

(30) Priority: 23.08.2004 JP 2004242752; 27.08.2004 JP 2004248346
(43) Date of publication of application: 30.05.2007
(73) Proprietor: Olympus Corporation, Tokyo 192-8507 (JP)
(72) Inventor: FUJITA, Manabu, Tokyo 1910003 (JP); HIRAKAWA, Katsumi, 2291103 (JP)
(74) Representative: von Hellfeld, Axel
(86) International application number: PCT/JP2005/015289
(87) International publication number: WO 2006/022269

(56) References cited:
- JP-A- 2 080 037
- JP-A- 4 108 143
- JP-A- 2001 143 005

## Description

### TECHNICAL FIELD

The present invention relates to an image display apparatus, an image display method, and an image display program that display a series of images taken by imaging a desired subject in time series.

### BACKGROUND ART

In recent years, in the field of endoscope, a capsule endoscope that is a swallowable endoscope including an imaging function and a radio communication function has been proposed, and an intra-subject information acquiring system that is a capsule endoscope system using the capsule endoscope to acquire image data of body cavities has been developed. After the capsule endoscope is swallowed from the mouth of a subject for observation (examination) and until naturally discharged from a human body, the capsule endoscope functions to sequentially image body cavities at predetermined intervals, for example intervals of 0.5 second, while passing through the body cavities, for example, inside of organs of the subject, such as a stomach and a small intestine, according to the peristalsis thereof.

Image data taken by the capsule endoscope while moving inside the body cavities is sequentially transmitted to an external receiving device by radio communication.
The receiving device has a radio communication function and a memory function, sequentially receives image data from the capsule endoscope via a predetermined wave, and sequentially stores the received image data in a memory. Since the subject carries the receiving device, the subject can freely move during a period from swallowing the capsule endoscope and until it is naturally discharged. After the capsule endoscope is naturally discharged from the subject, a doctor or a nurse takes image data stored in the memory of the capsule endoscope in an image display apparatus to display intra-subject images, for example, images of organs, on a display of the image display apparatus based on the obtained image data. The doctor or the nurse can perform diagnosis using the images of organs or the like displayed on the display (for example, see Patent Document 1).

Generally, an image display apparatus used in the above intra-subject information acquiring system has a processing function of observing (examining) inside of the body of a subject based on intra-subject images taken by a doctor or a nurse using a capsule endoscope. For example, the image display apparatus can sequentially display intra-subject images taken at predetermined intervals, for example, at intervals of 0.5 second during the period from swallowing a capsule endoscope from the mouse of a subject and until it is naturally discharged in time series. By observing (examining) the displayed intra-subject images, a doctor or a nurse can select an image of a disordered site such as a bleeding site or a lesion (abnormality image), an image of a featured site (featured site image) of an organ, or the like from the displayed images. The image display apparatus can save and manage, an image selected by the doctor or the nurse as image of interest such as an abnormality image or a featured site image separately as a selected image. Thereby, the doctor or the nurse can acquire data useful for physiological studies or diagnosis of a disordered site such as a bleeding site or a lesion over all of digestive tracts of a human body including deeper portions of a body (a small intestine and the like) where a well-known ultrasonic endoscope or the like cannot reach.

US 5,697,885 discloses an explanatory diagram showing a displaying example of a monitor showing a reference image before variation begins, temporarily selected images selected for comparison and a displayed image now being displayed.

JP 2001-143005 (A) and JP H09-81646 (A) disclose medical images display screens.

Patent Document 1: Japanese Patent Application Laid-open No. 2003-19111

### DISCLOSURE OF INVENTION

### PROBLEM TO BE SOLVED BY THE INVENTION

However, the capsule endoscope images inside of the body of a subject in time series at predetermined time intervals, for example, at intervals of 0.5 second in a period from swallowing the capsule endoscope in a subject and until it is naturally discharged, for a long period of time, namely for about eight hours or more. Therefore, the image display apparatus eventually displays a great number of images taken by the capsule endoscope. In this case, screen display allowing an easy estimation of the correspondence of an image selected by a doctor or a nurse as an abnormality image, a feature site image, or the like to a portion (an organ) of the subject is not particularly taken into consideration in the above image display apparatus.

Meanwhile, there is a case that images are sequentially displayed on a display in an imaging order at a predetermined display rate, and the doctor or the nurse selects an image required for diagnosis from the images sequentially displayed. Conventionally, however, when the doctor or the nurse confirms a selected image on the display, it is necessary to perform a complicated process such that they search a desired selected image from a plurality of images stored to display the selected image on the display, which can hinder rapid diagnosis by the doctor or the nurse.

The present invention has been achieved in view of these circumstances, and a first object thereof is to provide an image display apparatus and an image display program in which an imaging position of a selected image can be easily estimated from a display screen, a second object thereof is to provide an image display apparatus, an image display method, and an image display program where rapid confirmation of a selected image performed by a user is made possible.

In order to solve the above problems and to achieve the objects, the present invention provides an image display apparatus having the features set out in claim 1.

The above objects are also solved with an image display program as defined in claim 7.

Further embodiments are indicated in the dependent claims.

### EFFECT OF THE INVENTION

According to the present invention, there can be achieved an effect that an image display apparatus and an image display program where a time scale indicating the imaging period of a series of images can be displayed and an index indicating a position of a selected image from the series of images on the time scale corresponding to an imaging time of the selected image so that an imaging position of the selected image can be easily estimated by visually confirming the index.

According to the present invention, there can be achieved an effect that an image display apparatus and an image display program where a route image showing an imaging route of a series of images can be displayed and an index indicating a position of an image selected from the series of images on the route image corresponding to an imaging position of the selected image so that an imaging position of the selected image can be easily estimated by visually confirming the index.

According to the present invention, there can be achieved an effect that rapid confirmation of an image selected by a user can be made possible in order to display the image selected from sequentially displayed in a predetermined display area in a predetermined selected image display area.

### BRIEF DESCRIPTION OF DRAWINGS

FIG. 1 is a schematic diagram schematically exemplifying one configuration example of an intra-subject information acquiring system using an image display apparatus according to a first embodiment of the present invention;
FIG. 2 is a block diagram schematically showing one configuration example of the image display apparatus according to the first embodiment of the present invention;
FIG. 3 is a schematic diagram specifically exemplifying a display screen of a display unit;
FIG. 4 is a flowchart exemplifying a process procedure for display-controlling an index indicating a position of a selected image on a time scale corresponding to imaging date and time of the selected image;
FIG. 5 is a flowchart exemplifying a process procedure for display-controlling a mark indicating an external body imaging period;
FIG. 6 is a flowchart exemplifying a process procedure for color-coding marks indicating periods where identical images continue for respective identical image groups to display-control the marks;
FIG. 7 is a schematic diagram for specifically explaining one display example of a search data file in a data list display area;
FIG. 8 is a schematic diagram for specifically explaining another display example of a search data file in the data list display area;
FIG. 9 is a schematic diagram for specifically explaining one display example of a search screen of an examination data file displayed in the data list display area;
FIG. 10 is a schematic diagram for specifically explaining one example of a display screen displayed according to driving control for writing an examination data file in recording media;
FIG. 11 is a schematic diagram for specifically explaining one example of a display screen displayed according to driving control for taking in various data from a receiving device;
FIG. 12 is a schematic diagram for specifically explaining one example of a display screen displayed according to driving control for initializing the receiving device;
FIG. 13 is a schematic diagram showing one specific example of a display screen for explaining an operation of a control unit when a log-off operation has been performed;
FIG. 14 is a schematic diagram exemplifying a display screen when an index indicating a position on a time scale with regard to a selected image only of a selected thumbnail has been displayed;
FIG. 15 is a schematic diagram exemplifying a display screen when an index is displayed only on a designated thumbnail;
FIG. 16 is a schematic diagram exemplifying a display screen when marks with identical mode have been displayed for respective thumbnails as an index;
FIG. 17 is a schematic diagram exemplifying a display screen when a reduced image of a thumbnail has been displayed as an index;
FIG. 18 is a block diagram schematically showing one configuration example of an image display apparatus according to a second embodiment of the present invention;
FIG. 19 is a schematic diagram specifically exemplifying a display screen when a schematic internal-body image has been displayed;
FIG. 20 is a schematic diagram showing an entire configuration of an intra-subject information acquiring system according to a third embodiment;
FIG. 21 is a block diagram showing a schematic configuration of an image display apparatus according to the third embodiment;
FIG. 22 is a flowchart showing an image display process procedure performed by a control unit shown in FIG. 21;
FIG. 23 is a flowchart showing a process procedure of an image selection process shown in FIG. 22;
FIG. 24 is a diagram showing one example of a display screen of a display unit shown in FIG. 21;
FIG. 25 is a diagram showing one example of a display screen of the display unit shown in FIG. 21;
FIG. 26 is a diagram showing one example of a display screen of the display unit shown in FIG. 21;
FIG. 27 is a diagram showing one example of a display screen of the display unit shown in FIG. 21;
FIG. 28 is a block diagram showing a schematic configuration of an image display apparatus according to a fourth embodiment;
FIG. 29 is a flowchart showing an image display process procedure performed by a control unit shown in FIG. 28;
FIG. 30 is a flowchart showing a process procedure of an image selection process shown in FIG. 29;
FIG. 31 is a diagram showing one example of a display screen of a display unit shown in FIG. 28;
FIG. 32 is a configuration diagram showing a configuration of a computer system using any one of the first to the fourth embodiments; and
FIG. 33 is a block diagram showing a configuration of a main body in the computer system shown in FIG. 32.

### EXPLANATIONS OF LETTERS OR NUMERALS

- 1: Subject
- 2: Capsule endoscope
- 3: Receiving device
- 4, 21: Image display apparatus
- 5: Portable recording medium
- 6a to 6h: Receiving antenna
- 10: Input unit
- 11: Display unit
- 12: Reader/writer
- 13: External communication I/F
- 14: Storage unit
- 15, 22: Control unit
- 100: Main-image display area
- 101: Main image
- 110: Data list display area
- 111: Search condition input column
- 112: Search start icon
- 120: Subimage display area
- 121: Scroll
- 130: Play operation icon group
- 131: Play icon
- 132: Frame play icon
- 133: Fast play icon
- 134: Reverse play icon
- 135: Previous frame play icon
- 136: Fast reverse play icon
- 140: Time bar
- 141, 211: Slider
- 142: External body image bar
- 143, 144: Identical image bar
- 150: Number-of-display screen icon group
- 160: Examination date icon
- 161: Patient name icon
- 162: Search icon
- 163: Initialization icon
- 164: Download icon
- 165: Patient information icon
- 166: Option icon
- 167: Report icon
- 168: Exit icon
- 169: Log-off icon
- 170: Status display area
- 180: Bookmark
- 200a to 204a, 200b to 204b: Mark
- 210: Schematic internal-body image
- 302: Receiving device
- 302a: Receiving jacket
- 302b: External device
- 304, 504: Image display apparatus
- 305: Portable recording medium
- 320, 520: Input unit
- 321: Selection information input unit
- 330: Display unit
- 340: Storage unit
- 350, 550: Control unit
- 352: Selected image extracting unit
- 353: Reduced image generator
- 354: Timer
- 355, 555: Image display controller
- 323: Cursor
- 324: Play button
- 325: Stop button
- 332: Scroll bar
- 333: Slider
- 522: Restart information input unit
- 400: Computer system
- 401: Main body
- 402: Display
- 402a: Display screen
- 403: Keyboard
- 404: Mouse
- 405: Modem
- 406: Local area network or wide area network (LAN/WAN)
- 407: Public line
- 408: Flexible disk (FD)
- 409: CD-ROM
- 411: Another computer system (PC)
- 412: Server
- 413: Printer
- 421: CPU
- 422: RAM
- 423: ROM
- 424: Hard disk drive (HDD)
- 425: CD-ROM drive
- 426: FD drive
- 427: I/O interface
- 428: LAN interface
- L1 to L5: Line
- MP: Mouse pointer
- Q1 to Q4: Subimage mark
- RM1 to RM5: Report mark
- SP1 to SP5: Thumbnail
- SPla to SP5a: Reduced image

### BEST MODE(S) FOR CARRYING OUT THE INVENTION

Exemplary embodiments of an image display apparatus, an image display method, and an image display program according to the present invention will be explained below in detail with reference to the accompanying drawings. An image display apparatus used in an intra-subject information acquiring system which is a capsule endoscope system that acquires intra-subject information, for example, image data, using a capsule endoscope will be explained below as one example of an image display apparatus according to the present invention, however, the present invention is not limited to this example. Note that drawings are schematically shown, a relationship between thickness and width of each part, ratio of thickness of each part differs from actual cases, and needless to mention that also among drawings, in some parts, a relationship and ratio of sizes differ therebetween.

### First embodiment

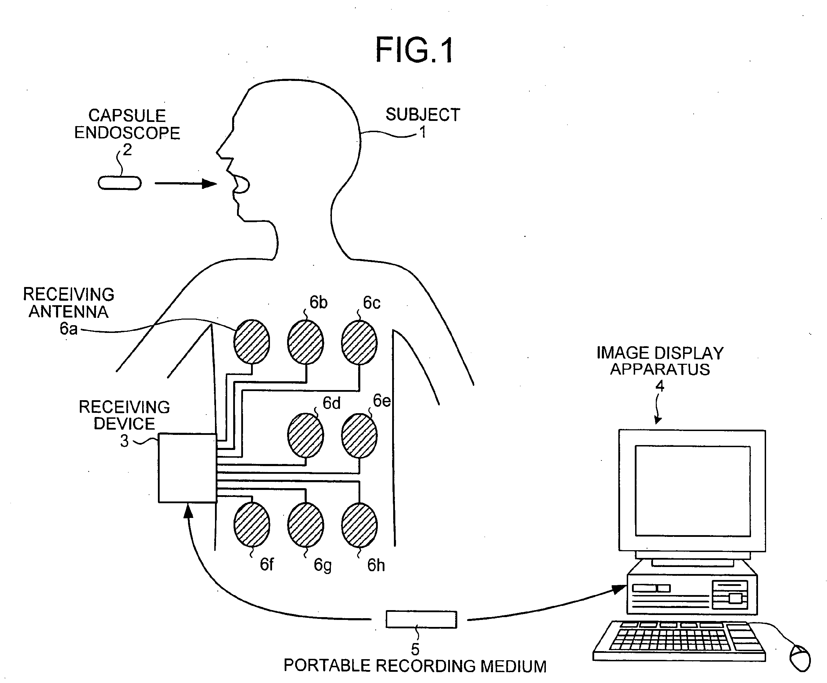

FIG. 1 is a schematic diagram schematically exemplifying one configuration example of an intra-subject information acquiring system using an image forming apparatus according to a first embodiment of the present invention. As shown in FIG. 1, the intra-subject information acquiring system includes a capsule endoscope 2 that moves along a passage route within a subject 1 and images an image inside the subject 1, a receiving device 3 that receives imaged data transmitted from the capsule endoscope 2, an image display apparatus 4 that displays images inside the subject 1 taken by the capsule endoscope 2 in time series, and a portable recording medium 5 for performing transmissions of information between the receiving device 3 and the image display apparatus 4.

The capsule endoscope 2 has an imaging function capable of imaging inside of the body of a subject and a radio communication function transmitting image data obtained by imaging the inside of the body of a subject to the outside. The capsule endoscope 2 passes through an esophagus inside the subject 1 according to swallow of the subject 1 to advance within a body cavity of the subject 1 according to peristalsis of a digestive tract. Simultaneously, the capsule endoscope 2 sequentially takes images in the body cavity of the subject 1. That is, the capsule endoscope 2 moves within the subject 1 according to peristalsis thereof during the period from swallowing the capsule endoscope 2 and until it is naturally discharged, and images inside the cavity body of the subject 1 at predetermined intervals, for example, at intervals of 0.5 second. In this case, the capsule endoscope 2 sequentially transmits image data inside the subject 1 to the outside.

The receiving device 3 has a radio communication function that receives image data from the capsule endoscope 2 via a predetermined radio wave transmitted and received between the capsule endoscope 2 and receiving antennas 6a to 6h. The receiving antennas 6a to 6h are realized, for example, by using loop antennas and receive radio waves transmitted for the capsule endoscope 2. As shown in FIG. 1, the receiving antennas 6a to 6h are disposed at a predetermined body surface of the subject 1, for example, positions corresponding to a passage route of the capsule endoscope 2 inside the subject 1. The receiving antennas 6a to 6h are electrically connected to the receiving device 3, respectively, and they transmit radio signals received from the capsule endoscope 2 to the receiving device 3. The receiving device 3 can sequentially receive image data inside the subject 1 from the capsule endoscope 2 via the receiving antennas 6a to 6h by sequentially receiving the radio signals.

The receiving device 3 has a memory function storing image data and the like and sequentially stores image data received from the capsule endoscope 2. In this case, the receiving device 3 determines imaging date and time of image data for each received image data, and associates imaging date and time information indicating the determined imaging date and time and each image data with each other. The receiving device 3 is preliminarily written with patient information and examination-specifying information associated to each imaging date and time information and each image data.

The receiving antennas 6a to 6h can be disposed at predetermined positions on a jacket that is worn by the subject 1. In this case, the receiving antennas 6a to 6h are disposed at predetermined positions on the subject 1 by the subject 1 wearing the jacket. Only disposition of a plurality of receiving antennas to the subject 1 is required and the number of receiving antennas to be disposed is not limited to eight. A plurality of receiving antennas are disposed on the subject 1, so that the receiving device 3 can receive image data from the capsule endoscope 2 via a receiving antenna at a position suitable for reception of a radio signal according to a position of the capsule endoscope 2 inside the subject 1.

The receiving device 3 can be attachably and detachably loaded with a portable recording medium 5 as a recording medium for storing image data received from the capsule endoscope 2. The portable recording medium 5 is attachable to and detachable from the receiving device 3 and the image display apparatus 4, and it has a structure for allowing outputting and recording of information to the receiving device 3 when loaded in the receiving device 3. Specifically, the receiving device 3 is loaded with the portable recording medium 5 to sequentially store image data inside the subject 1 sequentially received from the capsule endoscope 2 in the portable recording medium 5 while the capsule endoscope 2 is moving in the body cavities in the subject 1. In this case, as described above, the receiving device 3 associates each imaging date and time information and each image data with each other. The receiving device 3 stores patient information and examination-specifying information in the portable recording medium 5 together with each imaging date and time information and each image data.

After the capsule endoscope 2 is discharged from the subject 1, the portable recording medium 5 is taken out of the receiving device 3 to be loaded in the image display apparatus 4. As described above, the portable recording medium 5 can be attachably and detachably loaded in the image display apparatus 4, and it has a structure for allowing outputting and recording of information to the image display apparatus 4 when loaded in the image display apparatus 4. Accordingly, when the image display apparatus 4 is loaded with the portable recording medium 5, it can take in various data such as image data inside the subject 1 stored in the portable recording medium 5 by the receiving device 3.

Meanwhile, external communication interfaces for performing information communications between the receiving device 3 and the image display apparatus 4 are provided to the receiving device 3 and the image display apparatus 4, respectively. When the receiving device 3 and the image display apparatus 4 are electrically connected to each other via the external communication interfaces of the both, the image display apparatus 4 can take in various data such as image data inside the subject 1 described above from the portable recording medium 5 loaded in the receiving device 3.

As the portable recording medium 5, various recording media such as Compact Flash (registered trademark), a smart media, a memory stick, a multi-media card, or an SD memory card are used. By performing transmissions of data between the receiving device 3 and the image display apparatus 4 using such a portable recording medium 5, the subject 1 can freely move in a state that he/she carries the receiving device 3 even while the capsule endoscope 2 is moving inside the subject 1, which is different from a case that the receiving device 3 and the image display apparatus 4 are connected by wire using a communication cable or the like.

The image display apparatus 4 can be realized by a workstation or the like having processing functions for a doctor or a nurse to perform diagnosis based on images of organs or the like inside the subject taken by the capsule endoscope 2. For example, the image display apparatus 4 can sequentially display a series of images in time series at a predetermined intervals, for example, at intervals of 0.5 second during a period from swallowing the capsule endoscope 2 in the subject 1 and until it is naturally discharged, based on various data obtained from the receiving device 3 or the portable recording medium 5. The doctor or the nurse observes (examines) images inside the subject 1 sequentially displayed in the image display apparatus 4, so that they can select an abnormality image such as a bleeding site or a lesion, a featured site image of an organ, or the like from displayed images. The image display apparatus 4 can save and manage an image selected by the doctor or the nurse as an image of interest such as an abnormality image or a featured site image, as a selected image.

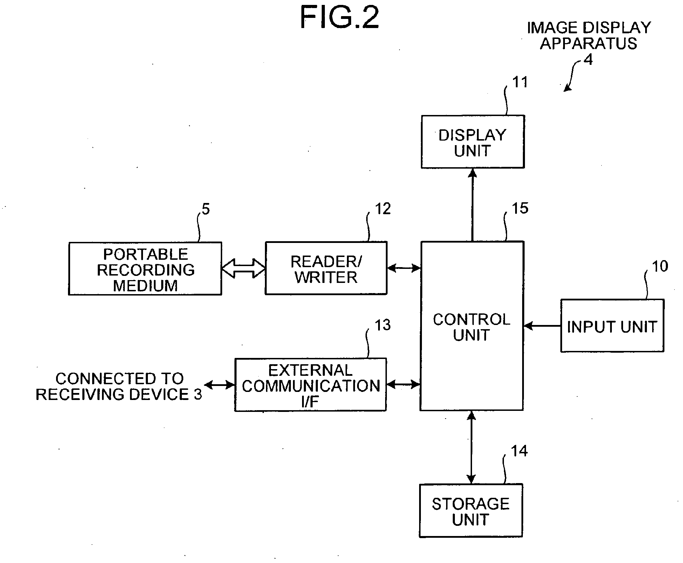

FIG. 2 is a block diagram schematically showing one configuration example of the image display apparatus 4. As shown in FIG. 2, the image display apparatus 4 has an input unit 10 that performs inputting of information required for observation (examination) inside the subject 1 or the like, a display unit 11 that displays images inside the subject 1 taken by the capsule endoscope 2 or the like on a screen, and a reader/writer 12 for taking in various data such as image data inside the subject 1 from the portable recording medium 5. The image display apparatus 4 has an external communication interface (I/F) 13 for performing information communication with the receiving device 3, a storage unit 14 that stores various data taken in from the receiving device 3 or the portable recording medium 5 or the like, a control unit 15 that performs driving control of respective configuration units in the image display apparatus 4, input/output control to information inputted/outputted to the respective configuration units, and information process.

The input unit 10 is realized by using a keyboard, a touch panel, a mouse, a track ball, or the like, or combination thereof, and it inputs information corresponding to an input operation performed by a user such as a doctor or a nurse in the control unit 15. For example, the input unit 10 inputs patient information such as name, sex, and age of a patient who is a subject, or a patient ID into the control unit 15 according to an input operation of the user. The input unit 10 inputs examination-specifying information such as an examination ID for specifying an examination to a subject or an examination date into the control unit 15 according to an input operation of the user. The patient information or the examination-specifying information is inputted as information required for performing examination to the subject. The input unit 10 inputs instruction information for performing driving control of respective configuration units in the image display apparatus 4 into the control unit 15 according to an input operation of a user. For example, the input unit 10 inputs instruction information for taking in image data or the like from the receiving device 3 or the portable recording medium 5, instruction information for displaying images taken from the capsule endoscope 2 on the display unit 11, or instruction information for selecting a desired image of interest such as an abnormality image or a featured site image from images displayed on the display unit 11 into the control unit 15.

The display unit 11 is realized by using various display such as a CRT display, a liquid crystal display, an organic EL display, or a plasma display, and it displays various information displayed and controlled by the control unit 15. The display unit 11 displays information inputted from the input unit 10 such as the patient information or examination-specifying information under control of the control unit 15. The display unit 11 sequentially displays images (for example, images inside the subject 1) based on image data taken in from the receiving device 3 or the portable recording medium 5 in time series under the control of the control unit 15. The display unit 11 displays information indicating a progress status of a process performed between the receiving device 3 and the image display apparatus 4, information indicating a state of the receiving device 3, information about an error which has been generated, and the like under the control of the control unit 15.

The reader/writer 12 can be attachably and detachably loaded with the portable recording medium 5. When the reader/writer 12 is loaded with the portable recording medium 5, it reads information stored by the receiving device 3, for example, image data acquired from - the capsule endoscope 2, or the like from the portable recording medium 5 and transfers the acquired image data and the like to the control unit 15 under the control of the control unit 15. In this case, the reader/writer 12 reads imaging date and time information, patient information, and examination-specifying information associated to the image data from the portable recording medium 5 together with the image data obtained from the capsule endoscope 2 and transfers the acquired imaging date and time information, patient information, and examination-specifying information to the control unit 15. Each image data transferred from the reader/writer 12 to the control unit 15 is associated with corresponding imaging data and time information. The reader/writer 12 has a function of recording information inputted from the control unit 15 and a function of performing formatting process to the loaded portable recording medium 5 under the control of the control unit 15.

The external communication I/F 13 is a communication interface for performing information communication with the receiving device 3. The control unit 15 can perform information communication with the receiving device 3 via the external communication I/F 13 when the external communication I/F 13 is electrically connected to the receiving device 3. For example, the control unit 15 can take in each image data obtained from the capsule endoscope 2 and each imaging date and time information in association with each other from the receiving device 3 or the portable recording medium 5 loaded in the receiving device 3. The control unit 15 can take in patient information and examination-specifying information from the receiving device 3 or the portable recording medium 5 loaded in the receiving device 3.

The storage unit 14 can be realized by using various IC memory such as a RAM (Random Access Memory), an EEPROM (Electronic Erasable Read Only Memory), or a flash memory, a hard disk, a Floppy (registered trademark) disk, an optical disk such as a CD (compact disk) or a DVD (Digital Versatile Disk), or an magnetic optical disk and a drive that can read or write data thereto. The storage unit 14 has a function of storing information inputted from the control unit 15 and a function of writing the information in various disks loaded under the control of the control unit 15. For example, the storage unit 14 stores patient information, examination-specifying information, or image data obtained from the capsule endoscope 2 or imaging date and time information of the image data or writes the same in various disks loaded under the control of the control unit 15. The storage unit 14 functions to read stored information and transfer the same to the control unit 15 under the control of the control unit 15.

The control unit 15 is realized by using a CPU (Central Processing Unit) that performs various process program, a ROM in which various process programs are stored in advance, and a RAM that stores arithmetic parameters for respective processes and the like. As described above, the control unit 15 functions so as to perform driving control of respective configuration units in the image display apparatus 4, input/output control to information inputted/outputted to/from the respective configuration units, and information process. For example, the control unit 15 performs driving control for taking in image data or the like from the receiving device 3 or the portable recording medium 5 or performs driving control for displaying an image based on the acquired image data on the display unit 11. The control unit 15 prepares an examination data file for collecting image data and imaging date and time information taken in from the receiving device 3 or the portable recording medium 5 for each patient information and for each examination-specifying information and stores the prepared examination data file in the storage unit 14 to save and manage the same. The control unit 15 saves and manages a desired image selected from displayed images through the input unit 10 used by a user as a selected image. The control unit 15 detects a state of the receiving device 3 via the external communication I/F 13 and stores patient information and examination-specifying information in the receiving device 3 according to the state of the receiving device 3 detected, thereby performing an initializing process for initializing the receiving device 3.

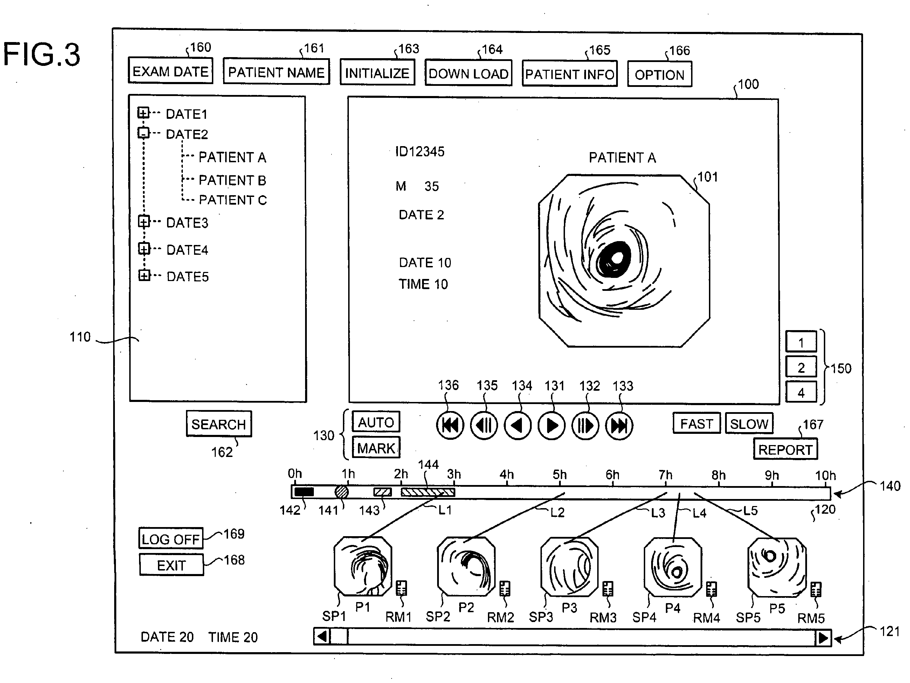

Next, a display screen displayed on the display unit 11 is specifically exemplified and an operation of the control unit 15 will be explained in detail. FIG. 3 is a schematic diagram specifically exemplifying a display screen of the display unit. That is, when a user performs a log-in operation for inputting a predetermined log-in password or the like using the input unit 10, the control unit 15 performs a predetermined log-in process based on information inputted from the input unit 10 according to the log-in operation to display a display screen exemplified in FIG. 3 on the display unit 11.

In FIG. 3, a main-image display area 100 that displays an image (main image) to be observed (examined) by a doctor or a nurse thereon, a data list display area 110 that displays the examination data file in a list, and a subimage display area 120 that displays a thumbnail corresponding to a desired image selected from the main screen displayed in the main-image display area 100 thereon are formed on the display screen of the display unit 11. A play operation icon group 130 including a plurality of icons for performing various play operations of a main image and a time bar 140 attached with a time scale indicating an imaging period of a series of images based on image data of the examination data file of an observed object are displayed near the main-image display area 100 on the display screen of the display unit 11.

A number-of-display screen icon group 150 for switching the number of main images simultaneously displayed in the main-image display area 100, an examination date icon 160 for switching a route of a hierarchical structure of the examination data file displayed in the data list display area 110 to an examination date, a patient name icon 161 for switching the route of the hierarchical structure of the examination data file displayed in the data list display area 110 to a patient name, and a search icon 162 for switching the data list display area 110 to a search screen of the examination data file are displayed on the display screen of the display unit 11.

Besides, an initialization icon 163 for performing an initializing process to the receiving device 3, a download icon 164 for taking in information such as image data from the receiving device 3 or the portable recording medium 5, an patient information icon 165 for displaying patient information in the main-image display area, an option icon 166 for performing an operation for writing the examination data file into an optical disk or an magnetic optical disk, a report icon 167 for preparing a finding, a diagnosis result, or the like for each main image, an Exit icon 168 for stopping an operation under execution or performing an operation for returning back to the last display screen, and a log-off icon 169 for performing a log-off operation are displayed on the display screen of the display unit 11. As shown in FIG. 3, Date20 (for example, year/month/day) indicating the current year, month, and date, and Time20 (for example, hour/minute/second) indicating the current time are displayed on the display screen of the display unit 11 in real time.

For example, a user performs a predetermined log-in operation using the input unit 10 to display the display screen exemplified in FIG. 3 on the display unit 11. Thereafter, the user selects an examination data file including image data to be observed (examined) from examination data files in the data list display area 110 using the input unit 10. In this case, the user can select the examination data file of the observation (examination) object by selecting a desired examination data file according to a key operation of the input unit 10 or moving a mouse pointer to a display position of the desired examination data file to perform a click operation for clicking the mouse button. The control unit 15 reads the selected examination data file from the storage unit 4 based on instruction information inputted from the input unit 10 according to a selection operation of the examination data file and can display (play) the image based on the image data included in the examination data file in the main-image display area 100 as the main image.

Next, when the user selects either one of play operation icons in the play operation icon group 130 using the input unit 10, the control unit 15 performs control for displaying a main image based on the image data of the examination data file in the main-image display area 100 to the display unit 11 according to instruction information inputted from the input unit 10. For example, as shown in FIG. 3, a play icon 131 for performing an operation for sequentially playing main images in a forward direction of time series, a frame play icon 132, a fast play icon 133, a reverse play icon 134 for performing an operation for sequentially playing main images in a direction reverse to the time series, a previous frame play icon 135, and a fast reverse play icon 136 are displayed as the play operation icon group 130. When a user performs a key operation or a click operation for selecting the play icon 131 using the input unit 10, the display unit 11 sequentially displays main images based on the image data of the examination data file selected by the user in the main-image display area 100 in a forward direction of the time series under the control of the control unit 15.

When the user performs a key operation or a click operation for selecting the frame play icon 132 using the input unit 10, the display unit 11 displays the next main image to this main image in the forward direction of the time series in the main-image display area 100 under the control of the control unit 15. That is, the display unit 11 repeats the operation for displaying the next main image to this main image in the forward direction of the time series under the control of the control unit 15 for each selection of the frame play icon 132 performed by the user. When the user performs a key operation or a click operation for selecting the fast play icon 133 using the input unit 10, the display unit 11 sequentially displays the main images in the forward direction of the time series in the main-image display area 100 at a play speed (a main image switching rate) faster than that in the case that the play icon 131 is selected under the control of the control unit 15.

On the other hand, when the user performs a key operation or a click operation for selecting the reverse play icon 134 using the input unit 10, the display unit 11 sequentially displays the main images based on the image data of the examination data file selected by the user in the reverse direction of the time series in the main-image display area 100 under the control of the control unit 15. When the user performs a key operation or a click operation for selecting the previous frame play icon 135 using the input unit 10, the display unit 11 displays a main image nest to the main image in the reverse direction of the time series (that is, a previous main image in the forward direction) in the main-image display area 100 under the control of the control unit 15. That is, the display unit 11 repeats an operation for displaying a main image next to the main image in the reverse direction of the time series (that is, a previous main image in the forward direction) in the main-image display area 100 under the control of the control unit 15 for each selection of the previous frame play icon 135 performed by the user. When the user performs a key operation or a click operation for selecting the fast play icon 136 using the input unit 10, the display unit 11 sequentially displays the main images in the reverse direction of the time series in the main-image display area 100 at a play speed (a main image switching rate) faster than the case that the reverse play icon 134 is selected under the control of the control unit 15.

As shown in FIG. 3, a "Fast" icon, a "Slow" icon, an "Auto" icon, and a "mark" icon are additionally displayed as a play operation icon group 130 on the display screen of the display unit 11. The "Fast" icon is an icon for performing an operation for increasing a play rate of main images displayed in the main-image display area 100. The "Slow" icon is an icon for performing an operation for decreasing a play rate of main images displayed in the main-image display area 100. When the user performs a key operation or a click operation for selecting the "Fast" icon using the input unit 10, the control unit 15 controls the display unit 11 to increase the play rate of the main images displayed in the main-image display area 100 to display them, and when the user performs a key operation or a click operation for selecting the "Slow" icon using the input unit 10, the control unit 15 controls the display unit 11 to decrease the play rate of the main images displayed in the main-image display area 100 to display them.

The "Auto" icon is an icon for performing an operation for automatically extracting an image of a bleeding site from all main images based on image data of the examination data file selected by the user. When the user performs a key operation or a click operation for selecting the "Auto" icon using the input unit 10, the control unit 15 automatically extracts an image of a bleeding site from all main images based on image data of the examination data file selected by the user or main images displayed in the main-image display area 100. When the control unit 15 extracts the image of the bleeding site, it applies a predetermined mark such as a flag indicating the image of the bleeding site as a bleeding site for each main image extracted automatically. When the user finds an image of a bleeding site from main images displayed in the main-image display area 100, the user can attach a predetermined mark such as a flag indicating the image of the bleeding site as a bleeding site for each main image extracted manually like the case that the image of the bleeding site is automatically extracted.

The "mark" icon is an icon for performing an operation for displaying an image of a bleeding site from all main images based on the image data of the examination data file selected by the user in the main-image display area 100. When the user performs a key operation or a click operation for selecting the "mark" icon using the input unit 10, the control unit 15 controls the display unit 11 to display only an image of a bleeding site extracted automatically or manually, as described above, from all main images based on the image data of the examination data file selected by the user in the main-image display area 100. Thereby, the user can observe (examine) only the image of a bleeding site from all the main images efficiently.

A user (namely, a doctor or a nurse) can find an abnormality image where a bleeding site or a disordered site such as a lesion has been imaged or a featured site image where a featured site of an organ has been imaged from all main images by observing (examining) main images displayed in the main-image display area 100 in the forward direction or the reverse direction of the time series, as described above. When an abnormality image or a featured site image is found, the user performs an operation for distinguishing the abnormality image or the featured site image from other main images using the input unit 10. Specifically, the user performs a key operation or a click operation for selecting the abnormality image or the featured site image displayed in the main-image display area 100 using the input unit 10. The control unit 15 saves and manages a desired main image (for example, an abnormality image or a featured site image) selected by the user as a selected image separately from the other main images and controls the display unit 11 to additionally display a thumbnail corresponding to the selected image in the subimage display area 120. When the user performs a key operation or a click operation for selecting the thumbnail in the subimage display area 120 using the input unit 10, the control unit 15 controls the display unit 11 to display a selected image specified by the selected thumbnail in the main-image display area 100 instead of a current main image based on instruction information inputted from the input unit 10 by the operation.

For example, when the user repeats an operation for selecting a desired number (for example, five) of main images using the input unit 10, the control unit 15 saves and manages respective selected main images as respective selected images and it controls the display unit 11 to additionally display thumbnails SP1 to SP5 respectively indicating the selected images in the subimage display area 120. Since the subimage display area 120 has a restriction regarding the display area, at most the predetermined number of thumbnails (for example, five) can be displayed. The control unit 15 controls the display unit 11 to display thumbnails of the number exceeding the restriction regarding the display area according to an operation of a scroll 121 using the input unit 10 performed by the user through switching to the subimage display area 120. When the user performs a key operation or a click operation for selecting, for example, thumbnails SP1 to SP5, using the input unit 10, the control unit 15 controls the display unit 11 to display selected images specified by the selected thumbnails SP1 to SP5 in the main-image display area 100 instead of current main images based on instruction information inputted from the input unit 11 according to the operation.

The control unit 15 can add a desired comment to each thumbnail in the subimage display area 120 based on information inputted from the input unit 10. For example, the control unit 15 can add respective comments of "P1", "P2", "P3", "P4", and "P5" to the thumbnails SP1 to SP5 based on information inputted from the input unit 10, as shown in FIG. 3. Thereby, the user can add an imaged organ name (for example, duodenum, jejunum, small intestine, or the like) or a desired comment specifying a thumbnail (for example, a bleeding site or a lesion) for each thumbnail in the subimage display area 120.

On the other hand, as described above, a time bar 140 attached with a time scale indicating an imaging period of a series of images based on an examination data file of an observed object is displayed on the display screen of the display unit 11. For example, a time scale indicating a time elapsed from an imaging start date and time of a series of images based on image data of an examination data file selected by a user is attached to the time bar 140 as the time scale indicating the imaging period of the series of images, as shown in FIG. 3. That is, the control unit 15 controls the display unit 11 to display the time scale indicating the elapsed time near the time bar 140 based on each imaging date and time information associated to each image data acquired for each patient information and for each examination-specifying information. The imaging start date and time is a date and time when the capsule endoscope 2 has started imaging of images in an examination data file of an observed object.

A slider 141 movable in a time-axis direction based on the time scale indicating the elapsed time is displayed on the time bar 140. The slider 141 is displayed as an index indicating a time elapsed from an imaging start date and time of a current main image displayed in the main-image display area 100. That is, the control unit 15 controls display of the display unit 11 such that the slider 14 moves toward a left end (a start position) or a right end (a termination position) of the time bar 140 or the slider 141 stops according to an operation of the play operation icon group 130 performed by the user. In this case, the control unit 15 controls display of the display unit 11 such that movement of the slider 141 and switching between main images displayed in the main-image display area 100 are simultaneously linked with each other, and imaging date and time of a current main image displayed in the main-image display area 100 and an elapsed time indicated by the slider 141 correspond to each other. For example, when the control unit 15 controls the display unit 11 to display a main image 101 where the year, month, and date of imaging is Date10 and imaging time is TimelO in a current main-image display area 100, the control unit 15 controls the display unit 11 to display the slider 141 at a position of the time scale on the time bar 140 indicating a time elapsed from imaging date and time (namely, imaging start date and time) of the oldest image data in the same examination data file as the main image 101.

Accordingly, a user (namely, a doctor or a nurse) can visually confirm a position indicated by the slider 141 of the time scale of the time bar 140 so that the user can easily recognize a time elapsed from an imaging start date and time of a current main image displayed in the main-image display area 100. A user (namely, a doctor or a nurse) can easily estimate the correspondence of the imaging portion of the current main image, namely, the current main image to an image showing a site (an organ) in the subject 1 based on the recognized elapsed time, a moving speed in the capsule endoscope 2 in the subject 1, or the like.

The control unit 15 controls the display unit 11 to sequentially display main images at respective imaging dates and times corresponding to respective positions on the time scale of the time bar 140 sequentially indicated by the slider 141 following the movement of the slider 141, namely, respective elapsed times in the main-image display area 100. Therefore, the user performs a key operation, a drag operation, or the like for moving the slider 141 to a desired position and displaying the same using the input unit 10 so that the user can display a main image at the desired imaging position in the main-image display area 100.

As described above, number-of-display screen icons 150 are displayed on the display screen of the display unit 11. When the user performs a key operation or a click operation for selecting either one of the number-of-display screen icons 150 using the input unit 10, the control unit 15 controls the display unit 11 to perform switching of the number-of-display screen of main images displayed in the main-image display area 100 according to the selected number-of-display screen icon 150. For example, when an icon designating the number-of-display screen of two from the number-of-display screen icons 150 is selected by the user, the control unit 15 controls the display unit 11 to be capable of sequentially displaying two continuous main images in the main-image display area 100, and when an icon designating the number-of-display screen of four from the number-of-display screen icons 150 is selected by the user, the control unit 15 controls the display unit 11 to be capable of sequentially displaying four main images in the main-image display area 100. When an icon designating the number-of-display screen of one from the number-of-display screen icons 150 is selected by the user, the control unit 15 controls the display unit 11 to be capable of displaying main images in the main-image display area 100 one by one.

As described above, the patient information icon 165 is displayed on the display screen of the display unit 11. When the user performs a key operation or a click operation for selecting the patient information icon 165 using the input unit 10, the control unit 15 controls the display unit 11 to perform switching to display of patient information corresponding to a current main image displayed in the main-image display area 100. In this case, the display unit 11 can display a patient name using characters of the number within a preset number of characters, a patient ID using alphameric characters or symbols of the number within a preset number of characters, patient sex indicating Male (M) or Female (F), patient age using numerals of the numbers within a preset digit number, and an examination date indicating the year, month, and date when the main image has been first observed (examined) as patient information. For example, when the patient information icon 165 is selected in a state that the main image 101 is being displayed in the main-image display area 100, as shown in FIG. 3, the display unit 11 displays the patient information corresponding to the main image 101, for example, "patient A" indicating a patient name, "ID12345" indicating a patient ID, "M" indicating patient sex, "35" indicating patient age, and "Date2" indicating an examination date. However, the Date2 indicates the year, month, and date which is an examination date of the main image.

When the patient information icon 165 is selected in a state that the patient information is being displayed in the main-image display area 100, the control unit 15 controls the display unit 11 to erase display of the patient information. The control unit 15 can control the display unit 11 to display an imaging date and time of a current main image (for example, DatelO indicating the year, month, and date of imaging and TimelO indicating the imaging time) like the patient information or it can control the display unit 11 to perform switching of displays of imaging dates and times of main images for each main image in synchronism with display control of main images.

As described above, the report icon 167 is displayed on the display screen of the display unit 11. When the user performs a key operation or a click operation for selecting the report icon 167 using the input unit 10, the control unit 15 performs control to the display unit 11 to display a report creation screen (not shown) that allows creation of a report such as a finding or a diagnosis result attached to a current main image displayed in the main-image display area 100 or a thumbnail selected from the subimage display area 120. The user can create a report including desired content on the report creating screen using the input unit 10. Thereafter, the control unit 15 saves and manages the report created by the user in association with the current main image or the selected thumbnail.

Simultaneously, the control unit 15 can perform display-control to attach a predetermined mark indicating that the report has been created (attached) to the current main image or the selected thumbnail. For example, as shown in FIG. 3, the control unit 15 controls the display unit 11 to display report marks RM1 to RM5 indicating that respective reports have been respectively attached to the thumbnails SP1 to SP5 near the thumbnails SP1 to SP5, respectively. The user performs a key operation or a click operation for selecting the report marks RM1 to RM5 or an operation for moving the mouse button to the report marks RM1 to RM5 of a selected object using the input unit 10 to display report content corresponding to selected report marks RM1 to RM5 on a screen.

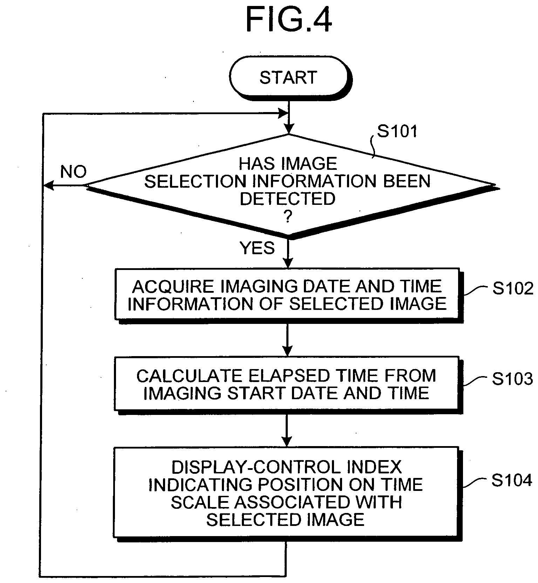

When a thumbnail corresponding to the image is additionally displayed in the subimage display area 120, the control unit 15 controls the display unit 11 to display, as an index indicating a position on the time scale of the time bar 140 corresponding to an imaging date and time of a selected image specified by the thumbnail, a line associating the thumbnail and the position on the time scale of the time bar 140 with each other. FIG. 4 is a flowchart exemplifying a process procedure for controlling display of an index indicating a position of a selected image on the time scale of the time bar 140 corresponding to imaging date and time. In FIG. 4, when the user performs a key operation or a click operation for selecting a desired main image using the input unit 10, as described above, image selection information indicating the selected desired main image is inputted into the control unit 15 from the input unit 10. In this case, the control unit 15 detects the image selection information (step S101, Yes) and saves and manages a main image selected and designated according to the detected image selection information as a selected image.

Next, the control unit 15 acquires imaging date and time information associated to the image data from the examination data file including imaged data of the selected image as imaging date and time information of the selected image (step S102). The control unit 15 acquires imaging date and time information of the oldest image data included in the examination data file as information indicating imaging start date and time (imaging start date and time information) in the examination data file. The control unit 15 calculates a time elapsed from the imaging start date and time until the selected image is taken based on the imaging date and time information and the imaging start date and time information (step S103).

Thereafter, the control unit 15 extracts a position on the time scale of the time bar 140 indicating the elapsed time obtained according to the process procedure at step S103, and determines the extracted position on the time scale as a position corresponding to the imaging date and time of the selected image. In this case, the control unit 15 stores and manages the extracted position on the time scale as the position on the time scale associated with the selected image. The control unit 15 performs display-control to the display unit 11 to additionally display a thumbnail corresponding to the selected image in the subimage display area 120 until this stage. Accordingly, when the control unit 15 determines the position on the time scale associated with the selected image, the control unit 15 performs display-control, to the display unit 11, of an index indicating the position on the time scale 140 associated with the selected image (step S104). In this case, the display unit 11 displays a line associating the thumbnail specifying the selected image and the position thereof on the time scale with each other as the index under the control of the control unit 15. As shown in FIG. 3, for example, the control unit 15 controls the display unit 11 to display lines L1 to L5 associating respective positions on the time scale caused to correspond to respective selected images respectively specified by the thumbnails SP1 to SP5 and the thumbnails SP1 to SP5 with each other as the indexes.

Thereafter, the control unit 15 repeats the process procedure of step S101 and steps subsequent thereto. Unless the user performs a key operation or a click operation for a selecting a desired main image using the input unit 11, the control unit 15 repeats the process procedure of step S101 without detecting the image selection information (step S101, No). That is, the control unit 15 constantly monitors whether the image selection information has been inputted from the input unit 10.

A user (namely, a doctor or a nurse) visually confirms the indexes exemplified by the lines L1 to L5 shown in FIG. 3 to know the correspondence of the thumbnail (for example, thumbnails SP1 to SP5) in the subimage display area 120 to a position on the time scale of the time bar 140 easily. Thereby, a user (namely, a doctor or a nurse) can easily recognizes the correspondence of the selected image specified by the thumbnail to the position on the time scale of the time bar 140 easily and can easily estimate the correspondence of the imaging position of the selected image, namely the selected image to an image of a site (an organ) in the subject 1 based on the recognized elapsed time indicated at the position on the time scale, a moving speed of the capsule endoscope 2 in the subject 1 and the like. When a user (namely, a doctor or a nurse) selects and designates an abnormality image such as a bleeding portion or a lesion as a selected image, they can estimate a site (an organ) in the subject 1 where a disordered site such as a bleeding portion or a lesion is present or concentrated easily. This is useful for the doctor or the nurse to diagnose a patient who is a subject.

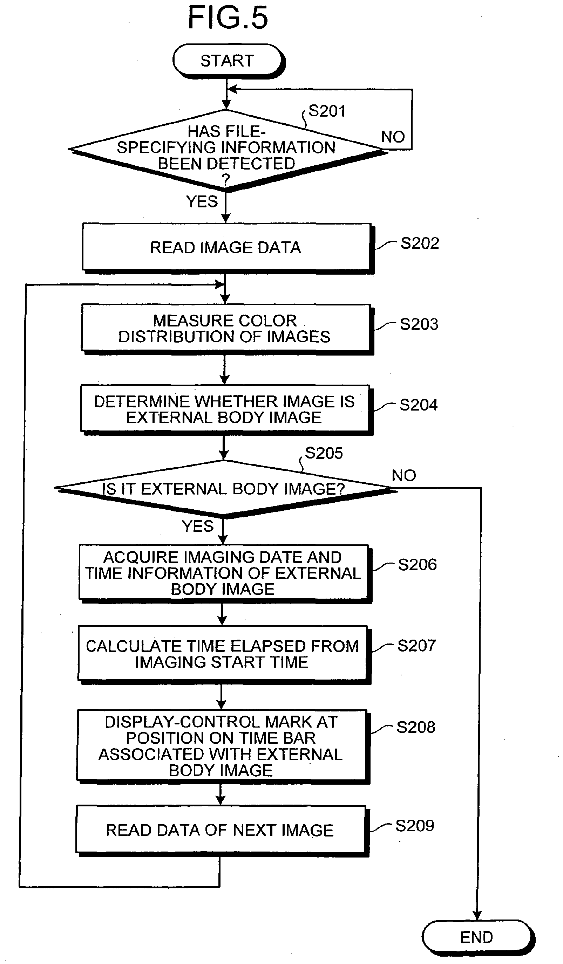

On the other hand, as shown in FIG. 3, an external body image bar 142 indicating a period (external body imaging period) where images (external body images) outside the subject 1 are taken by the capsule endoscope 2 is displayed on the time bar 140. FIG. 5 is a flowchart exemplifying a process procedure for controlling display of a mark indicating the external body imaging period on the time bar 140. The control unit 15 detects the external body imaging period where an external body image has been imaged by the capsule endoscope 2 and controls the display unit 11 to display a mark with a predetermined color indicating the detected external body imaging period on the time bar 140. That is, in FIG. 5, when the user performs a key operation or a click operation for selecting a desired examination data file from the examination data files in the data list display area 110 using the input unit 10, a file-specifying information for specifying the selected desired examination data file is inputted into the control unit 15 from the input unit 10. In this case, the control unit 15 detects the file-specifying information (step S201, Yes) and reads the oldest imaged image data (namely, image data of the imaging start date and time) from an examination data file specified by the detected file-specifying information (step S202).

Next, the control unit 15 uses the read image data to measure a color distribution of an image based on the image data (step S203) and determines whether the image based on the image data is an external body image based on the measurement result of the color distribution (step S204). When the control unit 15 determines that the image based on the image data is the external body image (step S205, Yes), it acquires imaging date and time information caused to correspond to the image data of the image determined as the external body image at step S203 from the examination data file specified in the file-specifying information detected at step S201 as imaging date and time information of the external body image (step S206). The control unit 15 acquires imaging date and time information of the oldest image data included in the examination data file as imaging start date and time information in the examination data file. The control unit 15 calculates a time elapsed from the imaging start date and time information until the external body image is taken based on the imaging date and time information and the imaging start date and time information (step S207).

Thereafter, the control unit 15 extracts a position on the time scale of the time bar 140 indicating the elapsed time acquired in the process procedure at step S207, and it determines the extracted position on the time scale as a position corresponding to the imaging day and time of the external body image. In this case, the control unit 15 stores and controls the extracted position on the time scale as a position on the time scale caused to correspond to the external body image. When the control unit 15 determines the position on the time scale caused to correspond to the external body image, it performs display-control of the mark with a predetermined color indicating the external body imaging period on the position on the time scale caused to correspond to the external body image (step S208) with respect to the display unit 11.

Next, the control unit 15 reads the next image data in the forward direction of the time series from the examination data file specified in the file-specifying information detected at step S201 (step S209), and it repeats the process procedure of step S203 and steps subsequent thereto. When the control unit 15 determines that the image of a determined object is not an external body image at step S204, the control unit 15 determines that the image of a determined object is an image (an internal-body image) obtained by imaging an inside of a subject 1 (step S205, No) to terminate the process procedure. That is, the control unit 15 can perform display-control of a mark with a predetermined color at each position on the time bar 140 corresponding to the imaging date and time for each external body image included in the examination data file of the observed object by performing the process procedure of steps S201 to S209 so that it can perform display-control of a continuous mark with the predetermined color indicating the external body imaging period of the examination data file of the observed object on the time bar 140. For example, the control unit 15 controls the display unit 11 to display an external body image bar 142 at a position of the time bar 140 exemplified in FIG. 3 as a continuous mark with a predetermined color indicating the external body imaging period of the examination data file of the observed object.

Unless the user performs a key operation or a click operation for selecting a desired examination data file using the input unit 10, the control unit 15 repeats the process procedure of step S201 without detecting the file-specifying information (step S201, No). That is, control unit 15 constantly monitors whether the file-specifying information has been inputted from the input unit 10.

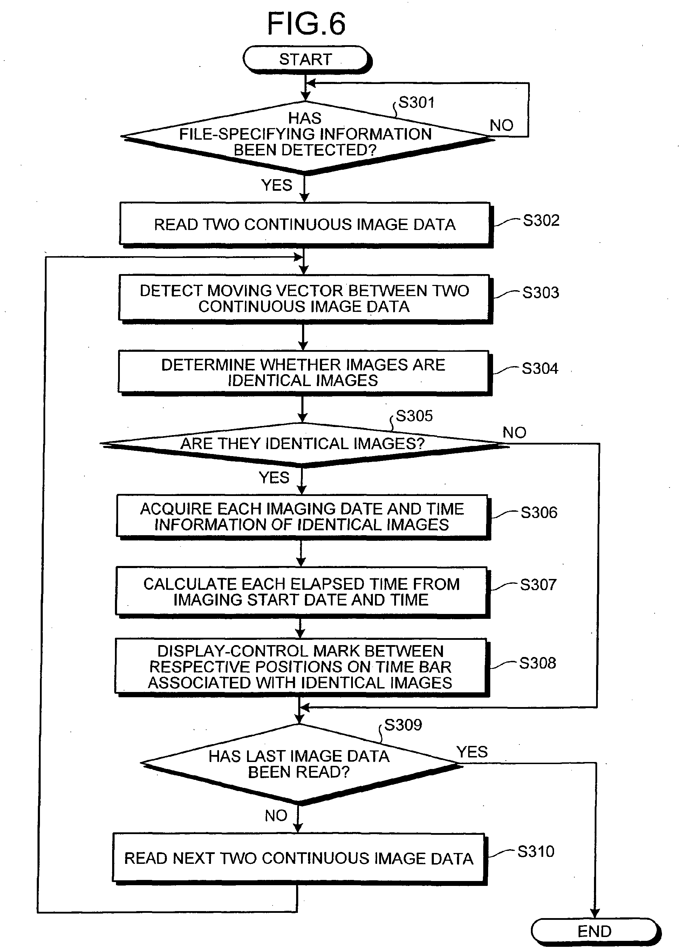

On the other hand, as shown in FIG. 3, the identical image bars 143, 144 indicating a period images that have been determined as identical images by the control unit 15 (the identical images) continue are color-coded and displayed on the time bar 140 for respective identical image groups. FIG. 6 is a flowchart exemplifying a process procedure for performing display-control of marks color-coded for respective identical image groups, indicating a period where identical images continue on the time bar 14. The control unit 15 detects a period where identical images continue in the examination data file selected by the user for each identical image and controls the display unit 11 to color-code and display a mark for each identical image on the time bar 140. In FIG. 6, when the user performs a key operation or a click operation for selecting a desired examination data file from examination data files in the data list display area 110 using the input unit 10, file-specifying information for specifying the selected desired examination data file is inputted into the control unit 15 from the input unit 10. In this case, the control unit 15 detects the file-specifying information (step S301, Yes) to read two image data continuous in the forward direction of the time series from the examination data file specified by the detected file-specifying information (step S302). It is desirable that, when the control unit 15 reads the two image data at step S302, it reads continuous image data from the oldest image data except for the image data of the external body image.

Next, the control unit 15 uses the read two continuous image data to detect a motion vector between respective images based on the two image data (step S303) and determines whether the respective images based on the two image data are the identical images based on the detected motion vector (step S304). When the control unit 15 determines that the respective images based on the two image data are the identical images (step S305, Yes),the control unit 15 acquires respective imaging date and time information caused to correspond to respective image data of the two images determined as the identical images at step S303 from the examination data file specified in the file-specifying information detected at step S301 (step S306). The control unit 15 acquires imaging date and time information of the oldest image data included in the examination data file as imaging start date and time information in the examination data file. The control unit 15 calculates respective elapsed times from the imaging start date and time until the two images are taken respectively based on respective imaging date and time information acquired and the imaging start date and time information (step S307).

Thereafter, the control unit 15 extracts respective positions on the time scale of the time bar 140 respectively indicating respective elapsed times obtained according to-the process procedure shown at step S307 and determines the respective extracted positions on the time scale as positions caused to correspond to the two images, namely, the identical images. In this case, the control unit 15 stores and manages the respective extracted positions on the time scale as respective positions on the time scale caused to correspond to respective imaging dates and times of the identical images. When the control unit 15 determines respective positions on the time scale caused to correspond to the identical images, it performs display-control of a mark with a predetermined color indicating a period where the identical images continue between respective positions on the time bar 140 corresponding to the respective determined positions on the time scale to the display unit 11 (step S308).

Next, the control unit 15 determines whether the last image data in the forward direction of the time series (namely, image data whose imaging date and time is the latest) from the examination data file specified in the file-specifying information detected at step S301 as image data for detecting the motion vector in the process procedure at step S303. When the control unit 15 determines that the last image data has not been read (step S309, No), it reads the next two image data in the forward direction of the time series from the examination data file specified in the file-specifying information detected at step S301 (step S310) to repeat the process procedure of step S303 and steps subsequent thereto.

When the control unit 15 determines that the two images of the determined object are not identical images at step S304, it determines that the two images are different from each other (step S305, No) to repeat the process procedure of step S309 and steps subsequent thereto. In this case, the control unit 15 saves and manages a plurality of images identical to an image just before the two images that have been determined as different images as one identical image group and controls the display unit 11 to display a mark indicating a period where the identical image groups are continued on the time bar 140 at a predetermined color. When the control unit 15 determines that the last image data has been read as the image data for detecting the motion vector at step S309 (step S309, Yes), it terminates the process procedure. That is, the control unit 15 can perform the process procedure of step S303 and steps subsequent thereto to approximately all image data in the examination data file specified in the file-specifying information detected at step S301.

Accordingly, the control unit 15 can color-code, for respective identical image groups, respective marks indicating respective imaging periods on respective positions on the time bar 140 corresponding to the respective imaging periods where identical images of the examination data file of an observed object continue to display-control the marks by performing the process procedure of steps S301 to S310. For example, the control unit 15controls the display unit 11 to display identical image bars 143, 144 with respective different colors on respective positions on the time bar 140 exemplified in FIG. 3 as marks color-coded for respective identical images and indicating the respective imaging periods where identical images continue. However, when the control unit 15 color-codes such marks indicating respective imaging periods of identical image groups for respective identical image groups to display-control the marks, it is desirable that colors different from that of the mark (for example, the external body image bar 142) indicating in the external body imaging period are used. Thereby, the control unit 15 can perform display-control to the display unit 11 to perform color-coding between respective marks indicating respective imaging periods of identical image groups and between the mark indicating the imaging period of the identical image group and the mark indicating the extracorporeal imaging period.

Unless the user performs a key operation or a click operation for selecting a desired examination data file using the input unit 10, the control unit 15 repeats the process procedure of step S301 without detecting the file-specifying information (step S301, No). That is, the control unit 15 constantly monitors whether the file-specifying information has been inputted from the input unit 10.

A user (namely, a doctor or a nurse) can recognize a period where the external body images continue in the imaging period shown on the time scale of the time bar 140 easily by visually confirming the mark exemplified on the external body image bar 142 in FIG. 3. The doctor or the nurse can recognize a period where identical images continue in the imaging period shown on the time scale of the time bar 140 easily by confirming the marks exemplified on the identical image bars 143, 144 in FIG. 3. Thereby, the doctor or the nurse can display a main image to be observed (examined) efficiently in the main-image display area 100, can find an abnormality image where a bleeding portion, a lesion, or the like has been imaged or the featured site image rapidly, and can perform a medical action such as diagnosis to a patient who is a subject efficiently.

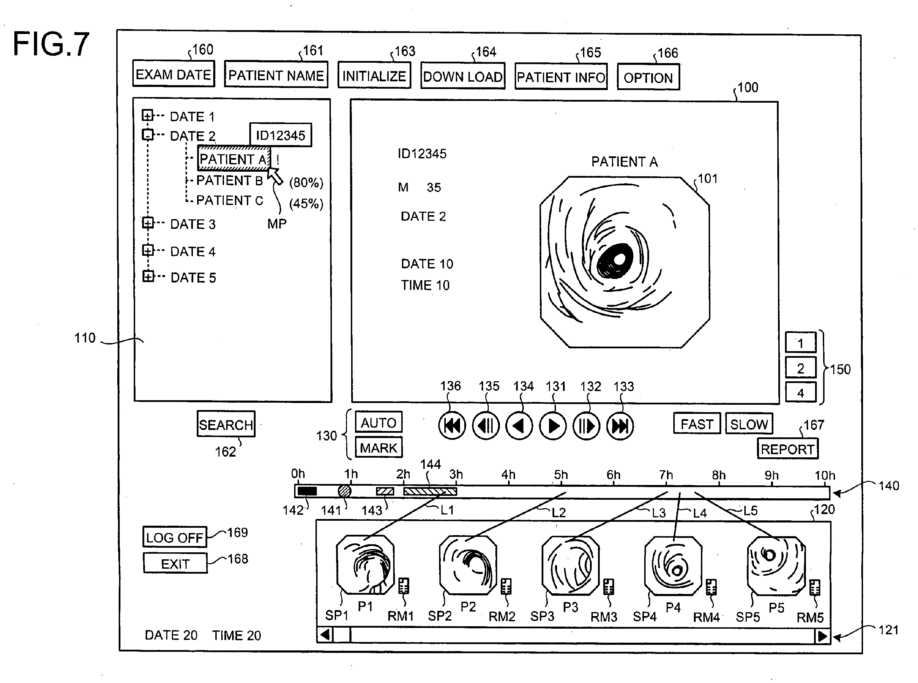

Next, the display-control regarding the data list display area 110 will be explained in details. FIG. 7 is a schematic diagram specifically exemplifying a display screen of the display unit 11, and is also a schematic diagram for specifically explaining one display example of a search data file in the data list display area 110. As described above, the control unit 15 prepares an examination data file obtained by collecting image data from the capsule endoscope 2 and the imaging date and time information for each patient information and for each examination-specifying information to save and manage the same. Accordingly, the control unit 15 can specify the examination data file by determining the patient information (for example, a patient name or a patient ID) and the examination-specifying information (for example, an examination date or an examination ID). In this case, the control unit 15 constructs a hierarchical structure (a tree structure) of the examination data file using the patient information and the examination-specifying information to control the display unit 11 to display a list of the examination data files based on the constructed tree structure in the data list display area 110.

For example, when the user performs a key operation or a click operation for selecting the examination date icon 160 using the input unit 10, as shown in FIG. 7, the control unit 15 utilizes the examination date (for example, Date1 to Date5) as a route to control the display unit 11 to display a list of the examination data file of the tree structure where a patient name (for example, patient A to patient C) follows a lower layer of the route, namely the examination date. The user can designate an examination date (namely, the examination-specifying information) and a patient name (for example, the patient information) of a desired examination data file by performing a key operation or a click operation for selecting the patient name following the lower layer of the examination date using the input unit 10. In this case, the control unit 15 receives information indicating the examination date and the patient name designated by the user from the input unit 10 as the file-specifying information.

The examination date used in the tree structure is the year, month, and date when a user (namely, a doctor or a nurse) has displayed the main image of the main-image display area 100 to observe (examine) the same. The user can designate the examination date arbitrarily by operating the input unit 10. The control unit 15 causes the examination date designated by the user to correspond to the examination data file. Alternatively, the control unit 15 can automatically set the year, month, and date where a main image has been displayed in the main-image display area 100 as an examination date of the examination data file of the main image. Date1 to Date5 exemplified in FIG. 7 indicate an examination date of each examination data file as date (for example, year/month/date).

The control unit 15 detects the number of main images displayed in the main-image display area 100 for each examination data file to perform display-control indicating an examination progress status according to the number of main images detected. For example, when the control unit 15 detects the number of displayed main images in the main-image display area 100 of all main images based on image data of the examination data file, the control unit 15 controls the display unit 11 to display the number of displayed main image to all the main image in the examination data file with percentage as display indicating the examination processing state. In this case, for example, as exemplified with display of "(80%)" near the display of "patient B" shown in FIG. 7, or display of "(45%)" near the display of "patient C", percentage display indicating the number of displayed main images to all the main images in the examination data file is displayed in the data list display area 110.

When the control unit 15 detects that all the main image based on the image data in the examination data file has already been displayed in the main-image display area 100, it determines the examination data file as observation (examination)-completed file and it performs display-control indicating that the examination data file has been observation (examination)-completed in the data list display area 110. As display indicating the observation (examination) completion, the control unit 15 can perform display-control for switching display modes like the examination data file specified by the examination date (Datel) and the patient name (patient A) in FIG. 7 or it can display-control a predetermined mark near a file name indicating the observation (examination)-completed examination data file. Alternatively, the control unit 15 can display-control percentage display (namely, 100%) indicating the number of displayed main images to all main images in the examination data file.

The control unit 15 confirms whether image data of an abnormality image taken by imaging a bleeding site, a lesion, or the like is present for each examination data file and performs display-control indicating the fact that the abnormality image is included (has been discovered) regarding the examination data file including the image data of the abnormality image. For example, when the control unit 15 detects the fact that an image data of an abnormality image is included in an examination data file specified by the examination date (Date2) and the patient name (patient A) shown in FIG. 7, it controls the display unit 11 to display a mark exemplified as "!" (exclamation mark) display in FIG. 7 near the file name of the examination data file (for example, near the display of "patient A") as a mark indicating inclusion of an abnormality image.

The control unit 15 can display-control not only "!" display exemplified in FIG. 7, but also a desired mark such as flag or asterisk as the mark indicating the fact that an abnormality image is included. The control unit 15 can perform display-control to conduct such switching that a display mode such as display color of a file name (for example, an examination date or a patient name) of an examination data file where the fact that image data of an abnormality image is contained has been detected, a display character style, or the like is distinguishable from another display mode as display indicating inclusion of an abnormality image.

When the user performs an operation for moving a mouse pointer MP to a position of the patient name display (for example, the display of "patient A") in the data list display area 110 to maintain the mouse pointer MP at the position of the patient name display for a predetermined period of time or more (for example, one second or more) using the input unit 10, as shown in FIG. 7, the control unit 15 controls the display unit 11 to display a patient ID (for example, patient ID "ID12345" of the patient A) of the patient name near the patient name display (for example, the display of "patient A"). Alternatively, when the user performs an operation for moving the mouse pointer MP to a position of the file name display (for example, the display of "patient A") of the examination data file in the data list display area 110 to maintain the mouse pointer MP at the position of the file name display for a predetermined period of time or more (for example, one second or more), the control unit 15 controls the display unit 11 to display a main image based on the image data in the examination data file in the main-image display area 100.

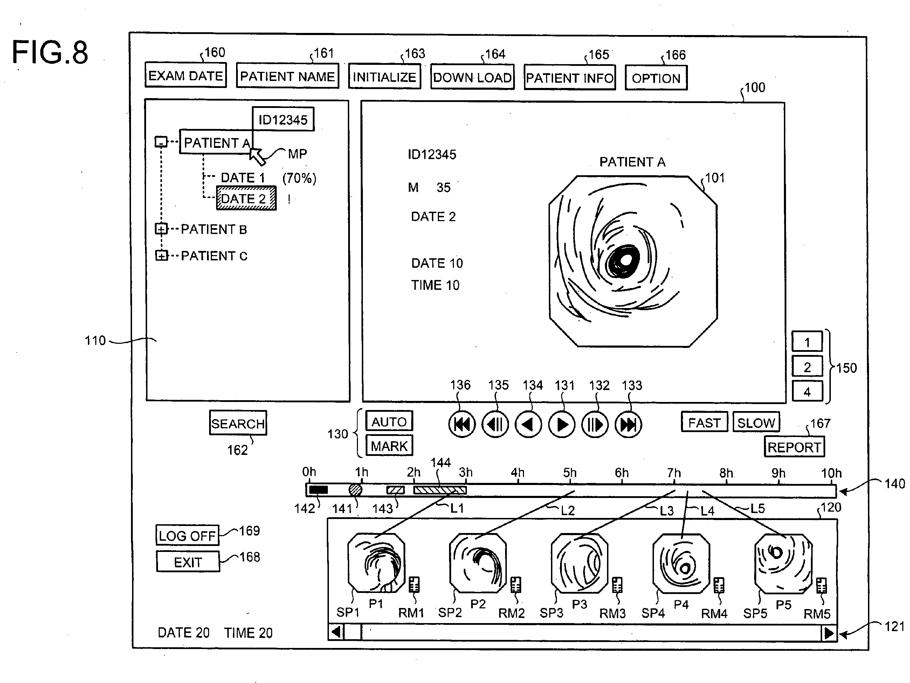

FIG. 8 is a schematic diagram specifically exemplifying a display screen of the display unit 11, and is a schematic diagram for specifically explaining another display example of the search data file in the data list display area 110. When the user performs a key operation or a click operation for selecting the patient name icon 161 using the input unit 10, as shown in FIG. 8, the control unit 15 utilizes the patient name (for example, Patient A to Patient C) as a route to control the display unit 11 to display a list of the examination data file of the tree structure where examination dates (for example, Date1 and Date2) follow a lower layer of the route, namely, the patient name. The user can designate the patient name (namely, the patient information) and the examination date (namely, the examination-specifying information) of a desired examination data file like the case of the operation for selecting the patient name following the lower layer of the examination date by performing a key operation or a click operation for selecting the examination date following the lower layer of the patient name using the input unit 10.

As shown in FIG. 8, when the control unit 15 performs display-control of list display of the examination data file based on the tree structure where the patient name is utilized as the route and the examination data follows the lower layer of the route, it performs, with respect to the display unit 11, display-control indicating an examination progress status for each examination data file corresponding to the number of displayed main images, display-control indicating the fact that image data of an abnormality image taken by imaging a bleeding site, a lesion, or the like is included for each examination data file, and display-control indicating a patient ID of a patient name indicated by the mouse pointer MP for a predetermined period of time or more like the case that the list display of the examination data file based on the tree structure'(namely, the tree structure where the examination date is utilized as the route and the patient name follows the lower layer of the route) is displayed and controlled. When the control unit 15 performs display-control of the list display of the examination data file based on the tree structure where the patient name is utilized as the route and the examination date follows the lower layer of the route, it controls the display unit 11 to display a main image based on the image data in the main-image display area 100 regarding the examination data file of the file name display (for example, the examination date) indicated by the mouse pointer MP for the predetermined period of time or more.

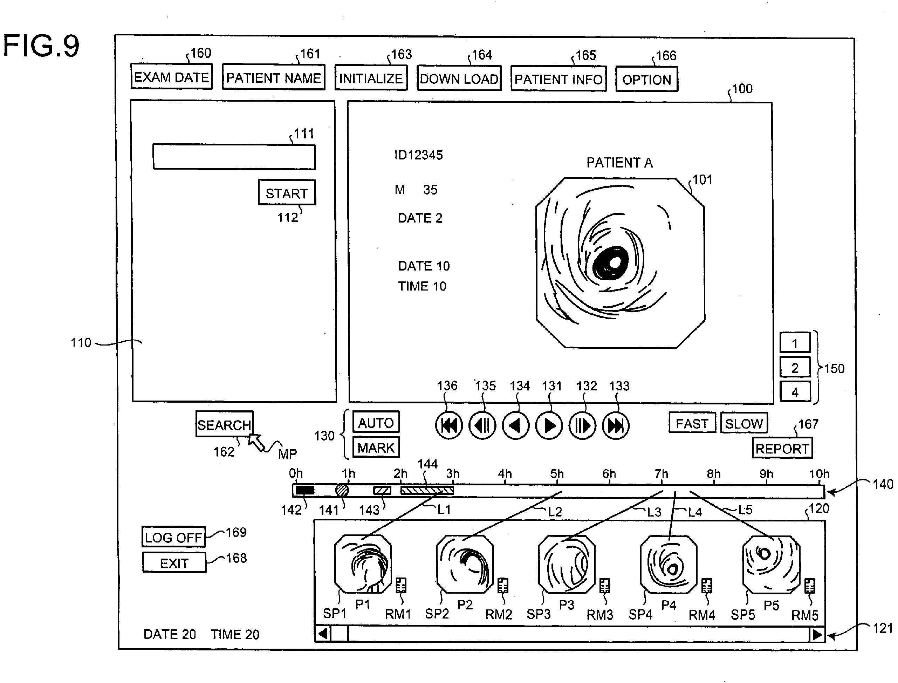

FIG. 9 is a schematic diagram specifically exemplifying a display screen of the display unit 11, and is a schematic diagram for specifically explaining one display example of a search screen of an examination data file displayed in the data list display area 110. When the user performs a key operation or a click operation for selecting a search icon 162 using the input unit 10, the control unit 15 controls the display unit 11 to display a search screen of the search data file in the data list display area 110. In this case, the control unit 15 controls the display unit 11 to display a search condition input column 111 and a search start icon 112 as the search screen as shown in FIG. 9.

The user performs an operation for inputting search conditions for searching a desired examination data file, for example, patient information (a patient name, a patient ID, patient age, patient sex, or the like) or examination-specifying information (an examination date, an examination ID, or the like) in the search condition input column 111 using the input unit 10. Next, the user performs a key operation or a click operation for selecting the search start icon 112 using the input unit 10. In this case, the control unit 15 searches an examination data file that satisfies all the inputted patient information and examination information, namely, satisfies AND condition of the inputted patient information and examination information based on the patient information and examination-specifying information inputted in the search condition input column 111. Thereafter, the control unit 15 controls the display unit 11 to display a list of the searched examination data files, namely examination data files satisfying the AND condition of the patient information and examination-specifying information inputted into the search condition input column 111 in the data list display area 110.

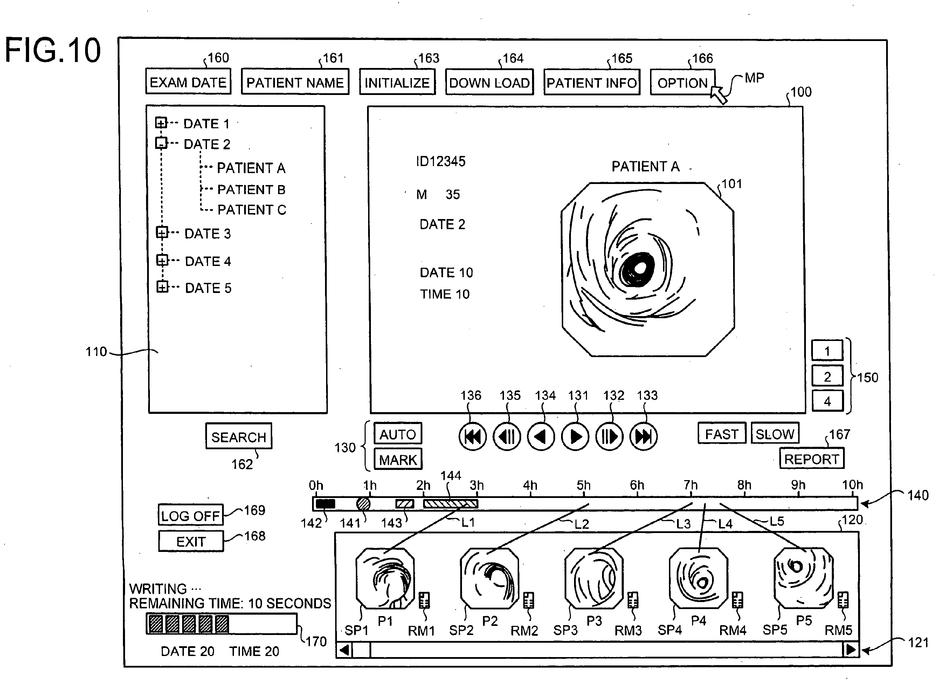

Next, display-control of a display screen according to driving control for writing examination data files in recording media loaded in the storage unit 14, such as a Floppy (registered trademark) disk, an optical disk, or a magnetic optical disk will be explained in details. FIG. 10 is a schematic diagram specifically exemplifying a display screen of the display unit 11, and is a schematic diagram for specifically explaining one example of the display screen displayed on the display unit 11 according to driving control for writing an examination data file in the recording media loaded in the storage unit 14. A user performs a key operation or a click operation for selecting an option icon 166 using the input unit 10 in a state that desired recording media has been attachably and detachably loaded in the storage unit 14. In this case, the control unit 15 performs driving control for writing a desired examination date file (for example, an examination data file selected by a user or all examination data files currently stored in the storage unit 14) in recording media loaded in the storage unit 14. When the control unit 15 writes an examination data file in the recording media it can perform a predetermined moving image compression process on an image data group of the examination data file to convert the examination data file to a moving image file.

Simultaneously, as shown in FIG. 10, the control unit 15 controls the display unit 11 to display a status display area 170 and controls the display unit 11 to sequentially display process progress marks indicating a process status of a write process of an examination data file to the recording media within the status display area 170 according to a progress status of the write process. The control unit 15 controls the display unit 11 to display a progress message indicating the progress status of the write process ("writing", "executing" or the like) a remaining time message indicating a remaining time until completion of the write process (for example, "Remaining time: 10 seconds") near the status display area 170. Thereafter, when the write process has been completed, the control unit 15 controls the display unit 11 to display a completion message (for example, "writing completed") indicating the fact that the write process has been completed near the status display area 170 instead of the progress message and remaining time message.

The control unit 15 performs such display-control to the display unit 11 so that when the process progress mark of the write process or each message regarding the progress status of the write process is separately displayed through pop-up, the user can omit a key operation or a click operation for closing the popped-up display content and can simplify an operation using the input unit 10.

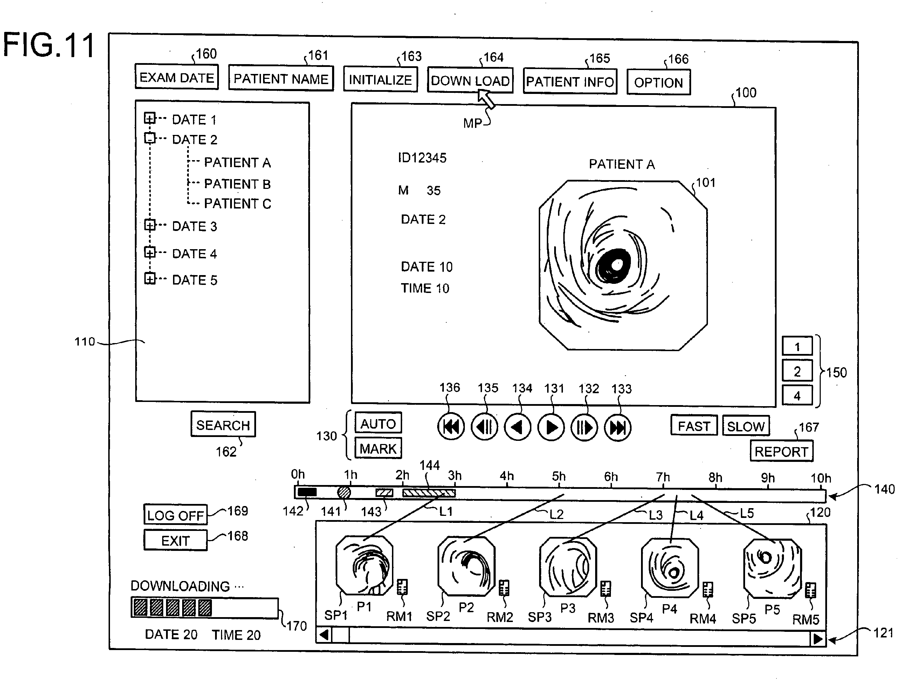

Next, display control of a display screen according to driving control for taking various data including the image data from the receiving device 3 electrically connected via the external communication I/F 13 will be explained in details. FIG. 11 is a schematic diagram specifically exemplifying a display screen of the display unit 11, and is a schematic diagram for specifically explaining one example of a display screen displayed on the display unit 11 according to driving control of taking in various data including image date from the receiving device 3 via the external communication I/F 13. The user performs a key operation or a click operation for selecting a download icon 164 using the input unit 10 in a state that the receiving device 3 and the image display apparatus 4 have been electrically connected to each other via the external communication I/F 13. In this case, when the receiving device 3 electrically connected via the external communication I/F 13 sequentially records image data or the like according to a legitimate process procedure, the control unit 15 performs driving control for taking in various data including the image data from the receiving device 3 or the portable recording medium 5 loaded in the receiving device 3.

Simultaneously, as shown in FIG. 11, the control unit 15 controls the display unit 11 to display the status display area 170, and controls the display unit 11 to sequentially display process progress marks indicating a process status of a process (a download process) taking in various data including the data from the receiving unit 3 within the status display area 170 according to the progress status of the download process. As shown in FIG. 11, the control unit 15 controls the display unit 11 to display a progress message ("downloading", "executing" or the like) indicating a progress status of the download process near the status display area 170. When the download process has been completed, the control unit 15 controls the display unit 11 to display a completion message (for example, "download completed") indicating the fact that the download process has been completed near the status display area 170 instead of the progress message of the download process. After completion of the download process, the control unit 15 performs driving control for erasing all image data stored in the receiving unit 3 to the receiving unit 3.

When the control unit 15 detects a failure of the storage unit 14 such as a failure of a hard disk during execution of the download process, it controls the display unit 11 to display an error message indicating the detected failure of the storage unit 14 within the status display area 170 or near the same. When the control unit 15 detects the fact that database in the storage unit 14 has been damaged during execution of the download process, the control unit 15 performs the display unit 11 to display an error message indicating detected error content within the status display area 170 or near the same. When the user performs an operation for moving the mouse button at a position of the status display area 170 using the input unit 10, the control unit 15 controls the display unit 11 to display an error message display history indicating a list of the past error messages displayed during the download processes in the time series order within the status display area 170 or near the same.

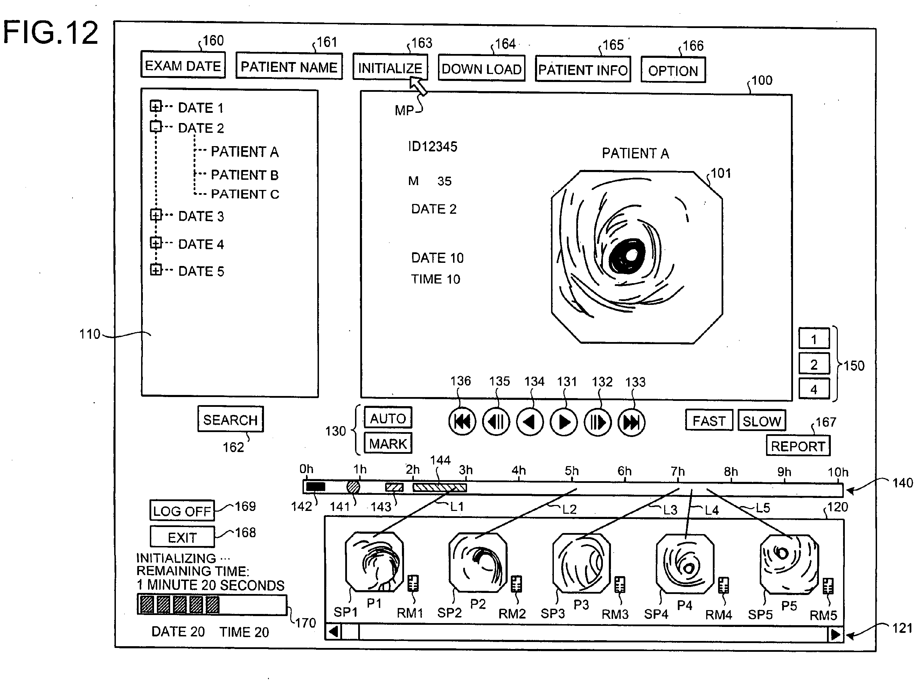

Next, display control of a display screen according to driving control of making the receiving unit 3 electrically connected via the external communication I/F 13 in an initial state (initializing the receiving unit 3) will be explained in details. FIG. 12 is a schematic diagram specifically exemplifying a display screen of the display unit 11, and is a schematic diagram for specifically explaining one example of the display screen displayed on the display unit 11 according to driving control of making the receiving unit 3 in the initial state via the external communication I/F 13. A user performs a key operation or a click operation for selecting an initialization icon 163 using the input unit 10 in a state that the receiving unit 3 and the image display apparatus 4 have been electrically connected to each other via the external communication I/F 13. In this case, the control unit 15 detects a state of the receiving unit 3 electrically connected via the external communication I/F 13 and it performs display-control of a status message indicating the detected state of the receiving unit 3 or performs an initialization process to the receiving unit 3.

For example, when the control unit 15 detects a state of the receiving unit 3 electrically connected via the external communication I/F 13 and determines that the detected state of the receiving unit 3 is not problematic, it performs driving control for making the receiving unit 3 in an initialization state by writing (overwriting) patient information (for example, a patient name, a patient ID, patient sex, and patient age) and examination-specifying information (for example, examination ID) in the receiving unit 3 via the external communication I/F 13. Thereby, the control unit 15 performs the initialization process to the receiving unit 13.

Simultaneously, as shown in FIG. 12, the control unit 15 controls the display unit 11 to display the status display unit 170, and controls the display unit 11 to sequentially display process progress marks indicating a process status of the initialization process to the receiving unit 3 within the status display area 170 according to the progress status of the initialization process. As shown in FIG. 12, the control unit 15 controls the display unit 11 to display a progress message ("initializing", "executing" or the like) indicating the progress process of the initialization process and a remaining time message (for example, "Remaining time: 1 minute 20 seconds") indicating a remaining time until completion of the initialization process near the status display area 170. Thereafter, when the initialization process has been completed, the control unit 15 controls the display unit 11 to display a completion message (for example, "initialization completed") indicating the fact that the initialization process has been completed near the status display area 170 instead of the progress message of the initialization process and the remaining time message.

On the other hand, when the control unit 15 determines that the detected state of the receiving unit 3 is problematic, it controls the display unit 11 to display a status message indicating the detected state of the receiving unit 3 without performing the initialization process to the receiving unit 3. In this case, the control unit 15 controls the display unit 11 to display, for example, a status message indicating a battery degradation (namely, battery shortage) in the receiving unit 3, a status message indicating the fact that image data remains in the receiving unit 3, or a status message indicating the fact that the storage unit in the receiving unit 3 or the portable recording medium 5 loaded in the receiving unit 3 reaches the end of life (namely, degraded to an extent that storage of image data is disturbed) as the status message.

When the user performs an operation for moving the mouse pointer to the position of the status display area 170 using the input unit 10, the control unit 15 controls the display unit 11 to display a status message display history indicating a list of the status messages displayed in past in the time series order within the status display area 170 or near the same.

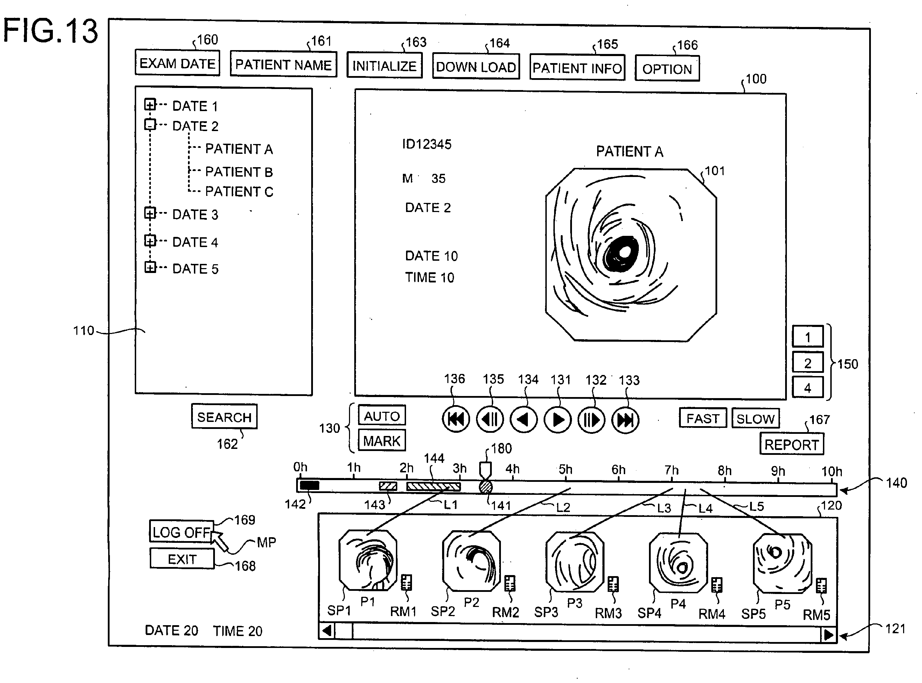

Next, an operation of the control unit 15 performed when a log-off operation has been performed by the user will be explained in details. FIG. 13 is a schematic diagram showing one specific example of a display screen of the display unit 11 for explaining an operation of the control unit 15 performed when a log-off operation has been performed by the user. When the user performs a log-off operation for returning a display screen of the display unit 11 after the log-in operation shown in FIG. 3 to a display screen before the log-in operation, the user performs a key operation or a click operation for selecting a log-off icon 169 using the input unit 10.

The control unit 15 performs a predetermined log-off process based on information inputted from the input unit 10 according to the log-off operation and controls the display unit 11 to display a display screen before the log-in operation from the display image after the log-in operation. In this case, the control unit 15 stores all display contents of the display screen displayed on the display unit 11 in the storage unit 14 at a time when the log-off operation has been performed by the user, and manages the all display contents of the display screen for each information specifying the user, for example, for each log-in password. Thereafter, when the user performs a log-in process again using the input unit 10, the control unit 15 performs a log-in process based on the log-in operation again and reads the all display contents in the display unit 11 from the storage unit 14 a time when the last log-off operation has been performed by the user to perform display-control to the display unit 11 to reproduce the read all display contents.

When the log-off operation is performed by the user, the control unit 15 stores an elapsed time indicated by the slider 141 on the time bar 140 at a time when the log-off operation has been performed in the storage unit 14 and manages the elapsed time for each information specifying the user, for example, for each log-in password. Thereafter, when the user performs a log-in operation again using the input unit 10, the control unit 15 performs a log-in process based on the log-in operation again and reads an elapsed time indicated by the slider 141 from the storage unit 14 at a time when the last log-off operation has been performed by the user to control the display unit 11 to display a bookmark 180 at a position above the time scale of the time bar 140 corresponding to the read elapsed time. Thereby, the control unit 15 can reproduce all display contents at the time of the log-off operation on the display unit 11, as exemplified in FIG. 13.

When the bookmark 180 is displayed on the time bar 140, the control unit 15 can cause the display unit to display a main image based on the oldest image data, namely, a leading image data in the forward direction of the time series from image data in the examination data file of the observed object in the main-image display area 100 at a time when the last log-off operation has been performed by the user. The user can recognize the main images observed (examined) before the time when the last log-off operation has been performed by the user easily by visually confirming the elapsed time indicated by the bookmark 180. The user can reproduce the main image (namely, the main image displayed at the time of the last log-off operation) corresponding to the elapsed time indicated by the bookmark 180 in the main-image display area 100 by performing an operation for moving the slider 141 to a position on the time scale of the time bar 140 indicated by the bookmark 180 using the input unit 11.

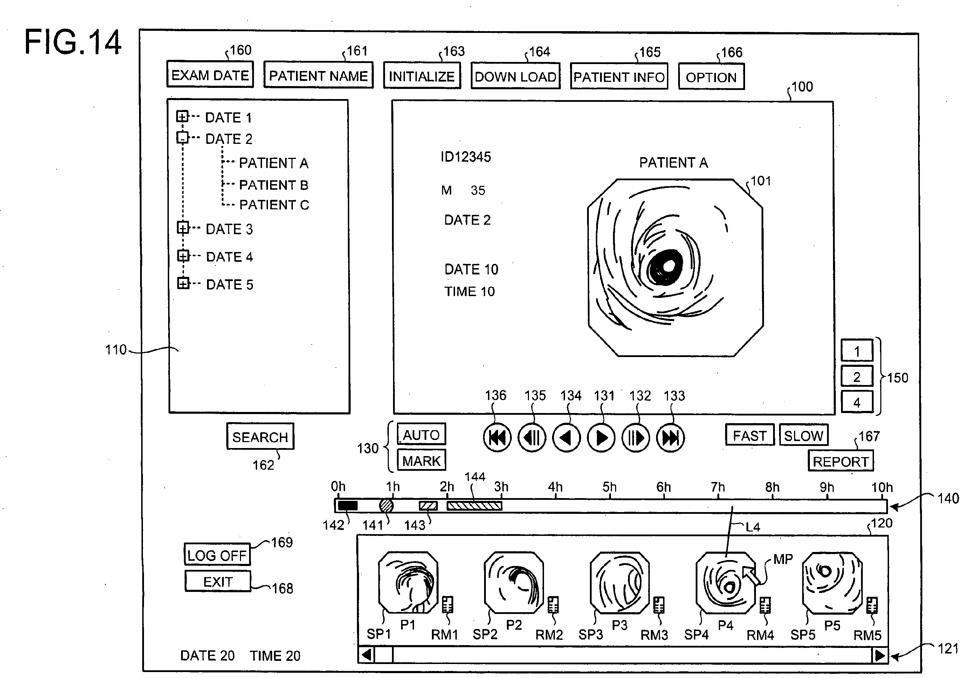

In the first embodiment of the present invention, indexes indicating a position on the time scale of the time bar 140 corresponding to imaging date and time of a selected image are displayed on all thumbnails displayed in the subimage display area 120, however, the present invention is not limited to the embodiment. The index can be displayed to only a thumbnail selected by the user. FIG. 14 is a schematic diagram exemplifying a display screen when an index indicating a position on a time scale of the time bar 140 with regard to an imaging date and time of a selected image only of a thumbnail selected by the user has been displayed.

The user performs a key operation or a click operation for selecting a desired thumbnail from thumbnails displayed in the subimage display area 120 using the input unit 10. In this case, as shown in FIG. 14, for example, the user can select a thumbnail SP4 by moving the mouse pointer MP to the thumbnail SP4. As shown in FIG. 14, the control unit 15 can display-control a line L4 that is an index indicating a position on the time scale of the time bar 140 corresponding to imaging date and time of a selected image only to the thumbnail SP4 selected by the user.

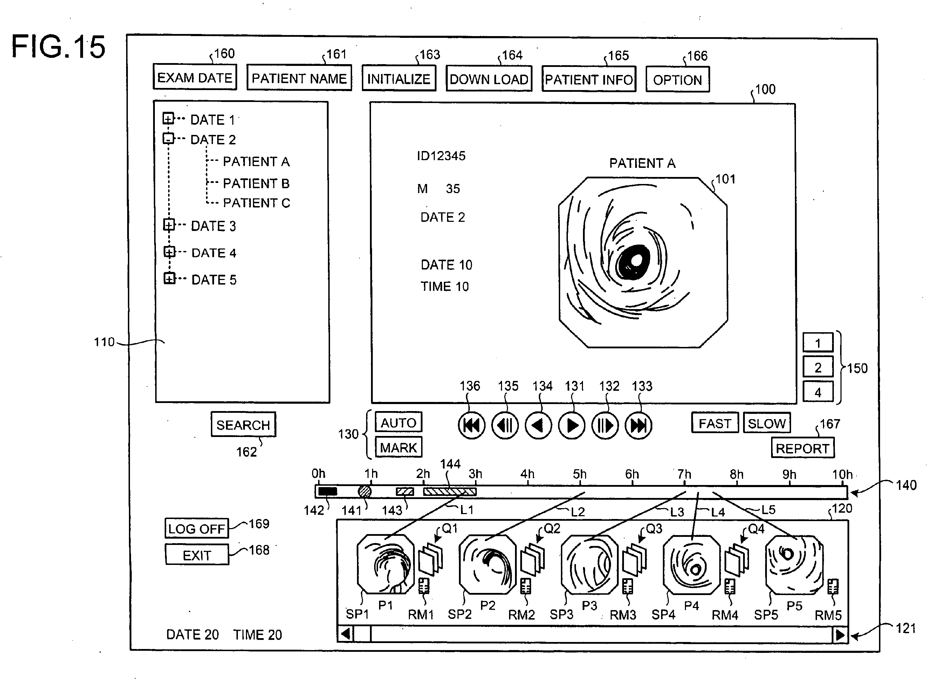

Further, in the first embodiment of the present invention, the indexes indicating a position on the time scale of the time bar 140 corresponding to imaging date and time of a selected image are displayed to all selected images from main images sequentially displayed, however, the present invention is not limited to the embodiment. The index can be displayed to only a selected image that has been designated with display of the index by the user. FIG. 15 is a schematic diagram exemplifying a display screen of the display unit 11 when the index is displayed to only the thumbnail of a selected image that has been designated with display of the index indicating a position on the time scale of the time bar 140 corresponding to imaging date and time of the selected image.

The user performs a key operation or a click operation for designating a desired selected image to be displayed with the index using the input unit 10. In this case, as shown in FIG. 15, the control unit 15 causes the display unit to display a thumbnail (for example, the thumbnails SP1 to SP5) of a selected image that has been designated with display of the index by the user in the subimage display area 120 and controls the display unit to display subimage marks Q1 to Q4 indicating one or more selected images which are not designated with display of the index in the subimage display area 120. In this case, the control unit 15 controls the display unit 11 to display the L1 to L5 which are the indexes regarding the thumbnails SP1 to SP5 of the selected images designated with display of the index, respectively. When the user sequentially performs a key operation or a click operation for selecting either one of the subimage marks Q1 to Q4 using the input unit 10, the control unit 15 can control the display unit 11 to sequentially display the selected images caused to correspond to the subimage marks selected by the user in the main-image display area 100.

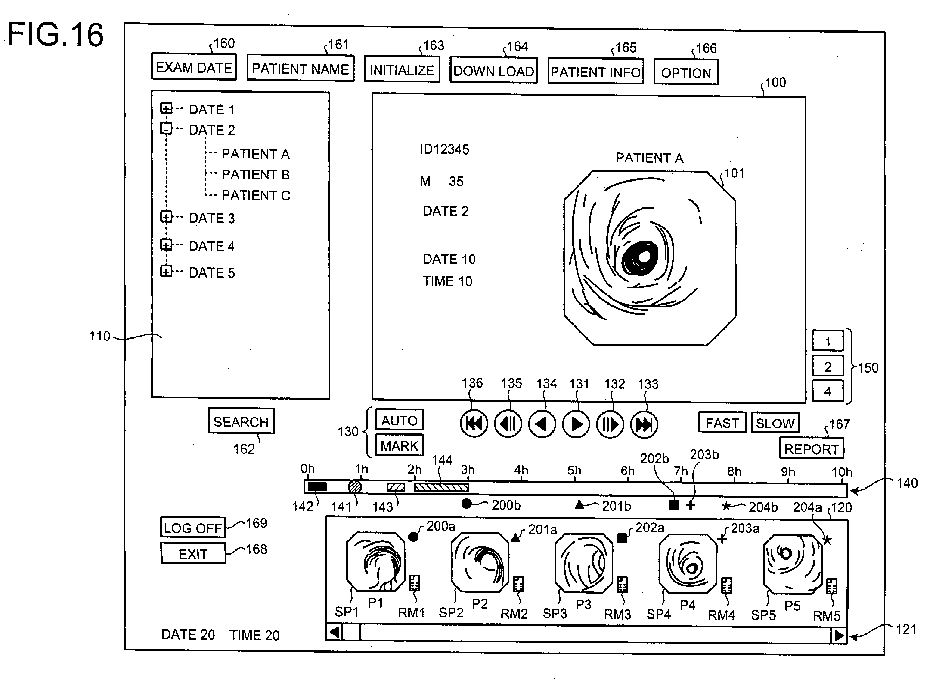

In the first embodiment of the present invention, the line associating a thumbnail specifying a selected image and a position on the time scale of the time bar 140 with each other is displayed as the index indicating a position on the time scale of the time bar 140 corresponding to imaging date and time of the selected image, however, the present invention is not limited to the embodiment. Marks with the same mode can be displayed to the vicinity of a thumbnail specifying a selected image and a position on the time scale of the time bar 140 as the indexes, respectively. FIG. 16 is a schematic diagram exemplifying a display screen of the display unit 11 when marks with the same mode are displayed for each thumbnail as an index indicating a position on the time scale of the time bar 140 corresponding to imaging date and time of a selected image.

When the user performs an operation for selecting and designating a desired selected image from main images sequentially displayed using the input unit 10, the control unit 15 performs a process procedure similar to step S101 to step S104 and controls the display unit 11 to display marks with the same mode near thumbnails and respective positions on the time scale of the time bar 140 for respective thumbnails instead of each line associating each thumbnail and each position on the time scale of the time bar 140 with each other. For example, as shown in FIG. 16, the control unit 15 controls the display unit 11 to display marks 200a to 204a near the thumbnails SP1 to SP5 and controls the display unit 11 to display marks 200b to 204b on respective positions on the time scale of the time bar 140 corresponding to imaging dates and times of respective selected images instead of the lines L1 to L5. The marks 200a to 204a have the same modes as the marks 200b to 204b, respectively. Accordingly, the user can easily confirm the correspondence of the respective selected images to the positions on the time scale of the time bar 140 by recognizing the marks 200a to 204a displayed near the thumbnails SP1 to SP5 and the marks 200b to 204b displayed at the respective positions on the time scale visually like the case of the lines L1 to L5.

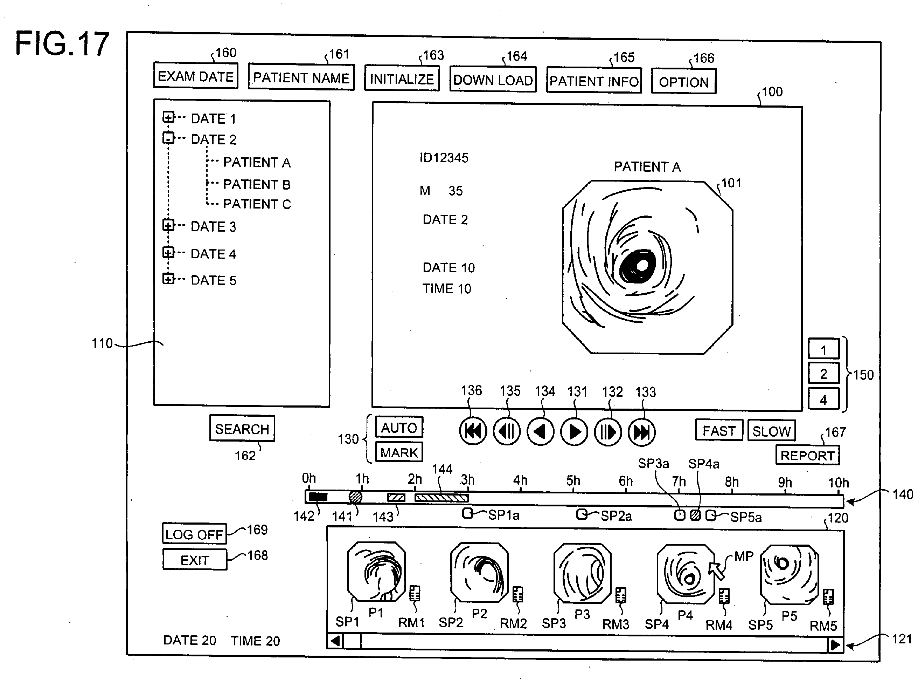

In the first embodiment of the present invention, the line associating a thumbnail specifying a selected image and a position on the time scale of the time bar 140 with each other is displayed as the index indicating a position on the time scale of the time bar 140 corresponding to imaging date and time of the selected image, however, the present invention is not limited to the embodiment. A reduced image of a thumbnail specifying a selected image can be displayed on a position on the time scale of the time bar 140, as the index. FIG. 17 is a schematic diagram exemplifying a display screen of the display unit 11 when a reduced image of a thumbnail has been displayed as an index indicating a position on the time scale of the time bar 140 corresponding to imaging date and time of a selected image.

When the user performs an operation for selecting and designating a desired selected image from main images sequentially displayed using the input unit 10, the control unit 15 performs a process procedure similar to step S101 to step S104 and controls the display unit 11 to display reduced images of respective thumbnail on respective positions on the time scale of the time bar 140 instead of each line associating each thumbnail and each position on the time scale of the time bar 140 with each other. For example, as shown in FIG. 17, the control unit 15 controls the display unit 11 to display respective reduced images SP1a to SP5a of the thumbnails SP1 to SP5 on respective positions on the time scale of the time bar 140 caused to correspond to selected images instead of the lines L1 to L5. In this case, when the user sequentially performs an operation for selecting either one of the reduced images SP1a to SP5a using the input unit 10, the control unit 15 can display the display unit 11 to sequentially display selected images specified by the thumbnails corresponding to the reduced images selected by the user on the main image display field 100.

When the user performs an operation for selecting a desired thumbnail using the input unit 10, the control unit 15 controls the display unit 11 to highlight the reduced image of the thumbnail selected by the user. For example, when the user performs an operation for moving the mouse pointer MP to the position of the thumbnail SP4 using the input unit 10, as shown in FIG. 17, the control unit 15 controls the display unit 11 to highlight the reduced image SP4a of the selected thumbnail SP4. Accordingly, the user can easily confirm the correspondence of the respective selected images to the positions on the time scale of the time bar 140 by recognizing the selected thumbnails SP1 to SP5 and the reduced images SP1a to SP5a highlighted visually like the case of the lines L1 to L5.

In the first embodiment of the present invention, respective thumbnails respectively corresponding to respective selected images selected by the user are additionally displayed on the subimage display field 120, however, the present invention is not limited to this embodiment. Respective subimages respectively corresponding to respective selected images can be additionally displayed on the subimage display field 120. For example, respective partial images partially clipped from respective selected images can be additionally displayed on the subimage display field 120, or images or schematic diagrams schematically showing respective selected images can be additionally displayed on the subimage display field 120.

In the first embodiment of the present invention, the time scale indicating a time elapsed from imaging start date and time about an examination data file selected by the user is attached to the time bar 140, however, the present invention is not limited to this embodiment. A time scale indicating imaging dates and times or imaging times of respective images about an examination data file selected by the user can be attached to the time bar 140.

In the first embodiment of the present invention, the imaging date and time information indicating imaging date and time of image data is taken in so as to correspond to the image data, however, the present invention is not limited to the embodiment. Time information corresponding to the imaging time of the image data can be taken in so as to correspond to the image data, elapsed time information indicating a time elapsed from imaging start of the image data can be taken in so as to correspond to the image data, or time information indicating imaging time of the image data can be taken in so as to correspond to the image data.

In the first embodiment of the present invention, the case that the list of the examination data file based on the tree structure constructed using a patient name and an examination date is displayed is shown, however, the present invention is not limited to the embodiment. A list of an examination data file based on a tree structure constructed by combining a patient ID or a patient name and an examination ID or an examination date can be displayed. When a list of an examination data file based on a tree structure using the patient ID is displayed, a patient name corresponding to the patient ID can be displayed near the patient ID by positioning the mouse pointer at the patient ID of the tree structure for a predetermined period of time or longer.

In the first embodiment of the present invention, the image display apparatus used in the intra-subject information acquiring system has been exemplified as one example of the image display apparatus according to the present invention, however, the present invention is not limited to the embodiment. An image display apparatus that allows screen display of a series of images taken in time series by an external imaging device can be adopted.

As explained above, in the first embodiment of the present invention, when the time scale indicating the imaging period of a series of main images taken in time series is displayed on a screen and a desired main image is selected from main images of an observed object, a subimage corresponding to the selected main image is additionally displayed and the index causing the subimage and the position thereof on the time scale to correspond to each other is displayed as the index indicating the position on the time scale corresponding to the imaging date and time of the selected main image. Therefore, the user can easily recognize periods in the imaging period where respective selected main images are present and time lengths of the periods by visually confirming respective indexes associating respective selected subimages and respective positions on the time scale with each other and can easily estimate respective imaging positions of the respective selected main images and distribution thereof by the visual recognition.

By using the image display apparatus according to the present invention, a user (namely, a doctor or a nurse) can easily estimate the correspondence between respective selected main images and sites (organs) inside the subject. Particularly, when images where a bleeding site or a disordered site such as a lesion has been imaged are selected from main images sequentially displayed, a site (an organ) inside the subject where the bleeding site or the disordered site such as a lesion is present or the bleeding sites or the disordered sites such as a lesion are concentrated can be estimated easily. This is useful for a doctor or a nurse to diagnose a patient who is a subject.

### Second Embodiment

Next, a second embodiment of the present invention will be explained in detail. In the first embodiment described above, the index associating a subimage such as a thumbnail corresponding to a selected image and a position thereof on the time scale with each other is displayed as the index indicating a position on the time scale of the time bar 140 corresponding to imaging date and time of the selected image. In the second embodiment, however, a schematic internal-body image schematically expressing a passage route of the capsule endoscope 2 inside the subject 1 is displayed and an index associating the subimage and a position on the schematic internal-body image with each other is displayed, instead of the index associating the subimage and the position thereof on the time scale.

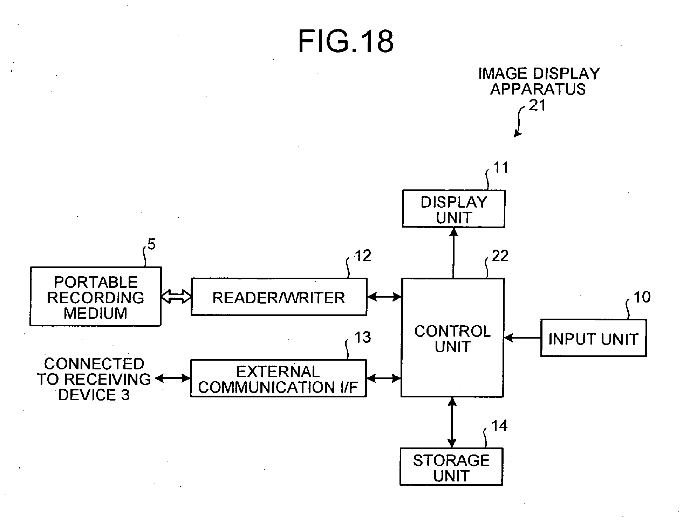

FIG. 18 is a block diagram schematically showing one configuration example of an image display apparatus according to the second embodiment of the present invention. The image display apparatus 21 includes a control unit 22 instead of the control unit 15 in the image display apparatus 4 according to the first embodiment. Other configurations are the same as those of the first embodiment, and like constituent elements are denoted with like reference letters or numerals.

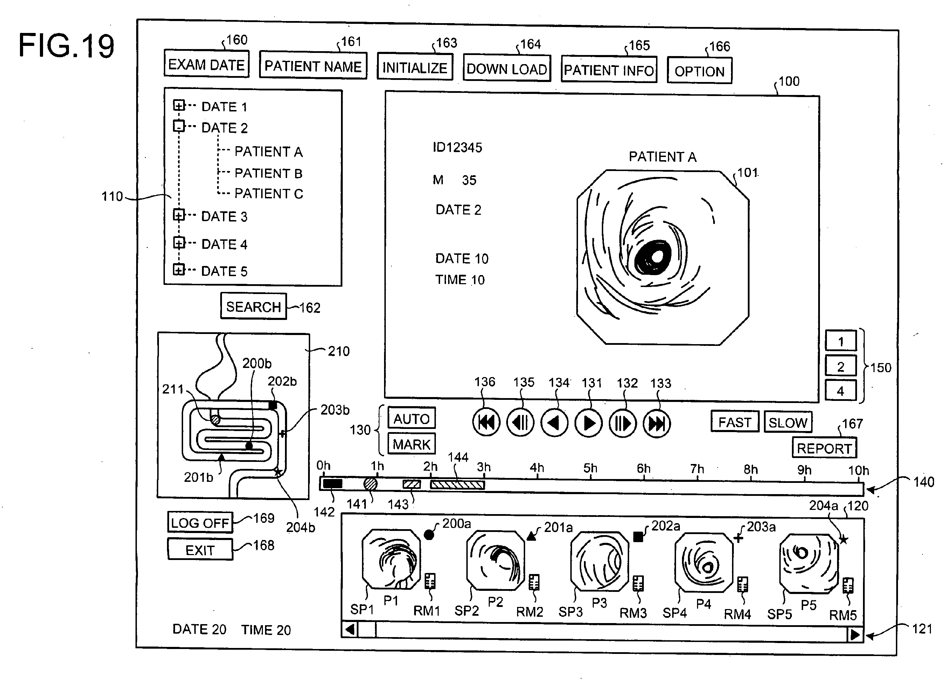

The control unit 22 functions similarly to the control unit 15, and performs display control regarding the schematic internal-body image and display control for associating a subimage (for example, a thumbnail) corresponding to a selected image and a position thereof on the schematic internal-body image with each other. FIG. 19 is a schematic diagram specifically exemplifying a display screen of the display unit 11 when a schematic internal-body image has been displayed. As shown in FIG. 19, the schematic internal-body image 210 schematically expresses a passage route (for example, an esophagus, a stomach, a small intestine, and a large intestine) of the capsule endoscope 2 inside the subject 1. Therefore, the schematic internal-body image 210 is displayed as a route image showing an imaging route of a series of images inside the subject 1 taken by the capsule endoscope 2.

A slider 211 movable along the imaging route is displayed in a range from a start position (an upper end) to an end position (a lower end) of the imaging route shown in the schematic internal-body image 210. The slider 211 is displayed as an index estimating an imaging position of a current main image displayed on the main image display field 100. In this case, the control unit 22 estimates an imaging position of the current main image based on a time elapsed from imaging start date and time of the examination data file to imaging date and time of the current main image and a moving speed of the capsule endoscope 2 inside the subject 1, and controls the display unit 11 to display the slider 211 at the position on the schematic internal-body image 210 corresponding to the estimated imaging position.

The control unit 22 performs display-control of the display unit 11 such that the slider 211 moves toward the start position (the upper end) or the end position (the lower end) of the imaging route or the slider 211 is stopped according to an operation of the play operation icon group 130 performed by the user. In this case, the control unit 22 performs synchronous linkage of the movement of the slider 211 and switching of main images to be displayed in the main-image display area 100 with each other and controls the display unit 11 such that the slider 211 indicates the imaging position of the current main image estimated based on the imaging date and time of the current main image displayed in the main-image display area 100 on the schematic internal-body image 210 similarly to the display-control of the slider 144.

For example, when an imaging position of a current main image displayed in the main-image display area 100 is estimated to be near the entrance of a small intestine, as shown in FIG. 19, the control unit 22 controls the display unit 11 to display the slider 211 at a position corresponding to the entrance of a small intestine on an imaging route displayed on the schematic internal-body image 210.

A user (namely, a doctor or a nurse) can easily estimate the imaging position of the current main image displayed in the main-image display area 100 and can easily estimate the correspondence of the current main image to a site (an organ) inside the subject 1 by visually confirming the position indicated by the slider 211 on the imaging route shown in the schematic internal-body image 21.

When the user performs a key operation, a drag operation, or the like for moving the slider 211 to a desired position on the imaging route and display the same thereat using the input unit 10, the control unit 22 can control the display unit 11 to sequentially display main images corresponding to respective positions sequentially indicated by the slider 211 on the imaging route in the main-image display area 100 following the movement of the slider 211 performed by the user, similarly to the case of the slider 141 described above. In this case, the time bar 140 and the slider 141 may not be displayed on the display screen of the display unit 11.

The control unit 22 performs a process procedure similar to steps S101 to S104 and controls the display unit 11 to display an index indicating a position of a selected image on the schematic internal-body image 210 corresponding to an imaging position instead of the index indicating the position of the selected image on the time scale of the time bar 140 corresponding to imaging date and time thereof. In this case, the control unit 22 estimates an imaging position of the selected image based on a time elapsed from imaging start date and time of a series of images including the selected image to the imaging date and time of the selected image. The control unit 22 controls the display unit 11 to display a index associating a thumbnail corresponding to the selected image and the position on the imaging route shown in the schematic internal-body image 210 with each other as the index indicating a position of the selected image on the schematic internal-body image 210 corresponding to the imaging position thereof.

For example, the control unit 22 causes the display unit 11 to display-control a mark 200a near the thumbnail SP1 and display-control a mark 200b with a mode identical to that of the mark 200a at a position associated with the thumbnail SP1 on the moving route of the schematic internal-body image 210 as indexes associating thumbnails corresponding to the selected images and positions of the imaging route shown-on the schematic internal-body image 210, as shown in FIG. 19. Similarly, the control unit 22 causes the display unit 11 to display-control marks 201a, 201b with an identical mode near the thumbnail SP2 and at a position associated with the thumbnail SP2 on the imaging route, and controls the display unit 11 to display control marks 202a, 202b with an identical mode near the thumbnail SP3 and at a position associated with the thumbnail SP3 on the imaging route. The control unit 22 causes the display unit 11 to display-control marks 203a, 203b with an identical mode near the thumbnail SP4 and at a position associated with the thumbnail SP4 on the imaging route, and controls the display unit 11 to display control marks 204a, 204b with an identical mode near the thumbnail SP5 and at a position associated with the thumbnail SP5 on the imaging route.

The control unit 22 controls the display unit 11 to display the marks 200b to 204b at respective positions on the imaging route respectively corresponding to imaging positions of respective selected images specified by the thumbnails SP1 to SP5 on the schematic internal-body image 210. Accordingly, a user (namely, a doctor or a nurse) can easily know positions associated with the thumbnails SP1 to SP5 on the imaging route by simultaneously visually confirming the marks 200a to 204a associated with the thumbnails SP1 to SP5 and the marks 200b to 204b displayed on respective positions on the imaging route shown in the schematic internal-body image 210. Thereby, a user (namely, a doctor or a nurse) can easily recognize positions associated with the respective selected images specified for respective thumbnails (for example, the thumbnails SP1 to SP5) in the subimage display area 120 on the imaging route shown in the schematic internal-body image 210 and can considerably easily estimate imaging positions of the respective selected images, namely, positions (organs) associated with the respective selected images inside the subject 1.

In the second embodiment of the present invention, regarding all thumbnails displayed in the subimage display area 120, the indexes indicating positions corresponding to imaging positions of selected images on the schematic internal-body image 210 have been displayed, however, the present invention is not limited to the embodiment. The index can be displayed regarding only a thumbnail selected by a user similarly to the case of the first embodiment.

In the second embodiment of the present invention, regarding all selected images, the indexes indicating positions on the schematic internal-body image 210 corresponding to imaging positions of the selected images have been displayed, however, the present invention is not limited to the embodiment. The index can be displayed regarding only a selected image whose index is designated by the user to be displayed similarly to the case of the first embodiment.

In the second embodiment of the present invention, marks with the same mode are placed near a thumbnail specifying a selected image and a position on the schematic internal-body image 210 as indexes indicating a position on the schematic internal-body image 210 corresponding to an imaging position of the selected image, however, the present invention is not limited to the embodiment. A line associating a thumbnail specifying a selected image and a position on the schematic internal-body image 210 with each other can be displayed as the index like the case in the first embodiment or a reduced image of a thumbnail corresponding to a selected image can be displayed on a schematic internal-body image 210.

Further, in the second embodiment of the present invention, an imaging position of a main image is estimated based on an elapsed time of imaging start date and time of a series of images to imaging date and time of the main image, however, the present invention is not limited to the embodiment. An imaging position of an image based on the capsule endoscope 2 from the receiving device 3 can be acquired as the imaging position of the main image. In this case, the control unit 22 can extract an imaging position of a selected image based on an imaging position acquired from the receiving device 3 and control the display unit 11 to display an index indicating a position on the schematic internal-body image 210 corresponding to the imaging position of the selected image like the second embodiment.

As described above, since the second embodiment of the present invention includes a configuration similar to that of the first embodiment, displays a route image showing a series of main images taken in time series on a screen, and when a desired main image is selected from main images of an observed object, additionally displays a subimage corresponding to the selected main image, and further displays an index associating the subimage and a position on a route image with each other as an index indicating a position of the route image corresponding to an imaging position of the selected main image, a user obtain operational effects similar to those in the first embodiment, and can estimate respective imaging positions of respective main images selected and a distribution thereof considerably easily by visually confirming respective indexes associating respective subimages and respective position thereof on the route image.

### Third Embodiment

Next, an image display apparatus according to a third embodiment of the present invention will be explained with reference to the drawings. An image display apparatus according to the third embodiment displays an image selected by a user from images sequentially displayed in a predetermined image display area in a predetermined selected image display area. In the description of the drawings, like parts are designated with like reference letters or numerals.

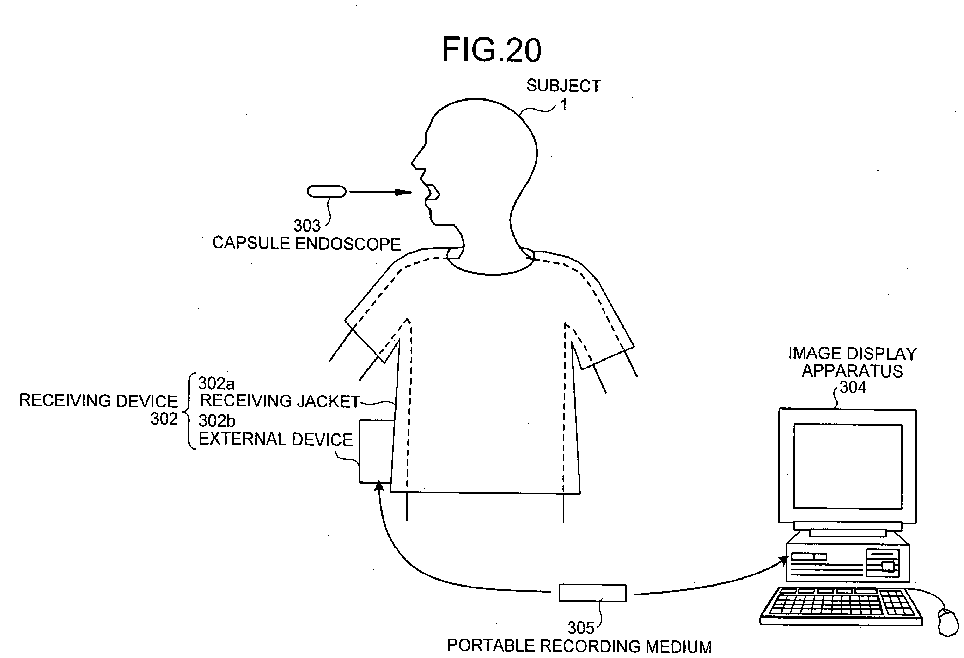

FIG. 20 is a schematic diagram showing an entire configuration of an intra-subject information acquiring system according to the third embodiment. As shown in FIG. 20, the intra-subject information acquiring system according to the third embodiment includes a receiving device 302 having a receiving function and a capsule endoscope 303 inserted in the body of the subject 1, and images an intra-subject image to transmit data to receiving device 302. The intra-subject information acquiring system includes an image display apparatus 304 displaying an intra-subject image based on the data received by the receiving device 302 and a portable recording medium 305 performing transmissions of data between the receiving device 302 and the image display apparatus 304. The receiving device 302 includes receiving jacket 302a worn by a subject 1 and an external device 302b performing a process of radio signal transmitted/received via the receiving jacket 302a and the like.

The image display apparatus 304 displays an intra-subject image taken by the capsule endoscope 303 and has a workstation or the like that performs image display based on data obtained by the portable recording medium 305. The image display apparatus 304 sequentially displays a plurality of still images taken by the capsule endoscope 303 in the imaging order. Images sequentially displayed are called "pseudo moving image".

The portable recording medium 305 is attachable to and detachable from the external device 302b and the image display apparatus 304, and it has a structure where outputting or recording of information can be performed at a time of loading to both the apparatus. Specifically, the portable recording medium 305 is loaded to the external device 302b to record data transmitted from the capsule endoscope 303 while the capsule endoscope 303 is moving in the body cavities in the subject 1. After the capsule endoscope 303 is discharged from the subject 1, namely, after imaging of insides of the subject 1 are completed, the portable recording medium 305 is taken out of the external device 302b, is loaded into the image display apparatus 304, and data recorded by the image display apparatus 304 is read. For performing transmissions of data between the external device 302b and the image display apparatus 304, a configuration where the external device 302b and the image display apparatus 304 is connected by a wire can be adopted instead of the configuration where transmissions of data are performed by the portable recording medium 305 such as a Compact Flash (registered trademark) memory.

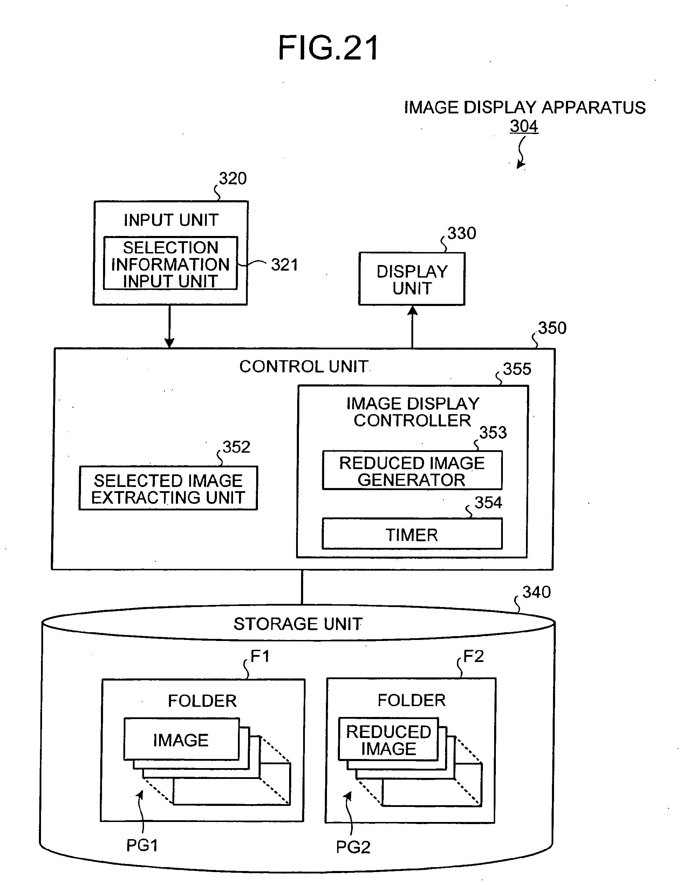

The image display apparatus 304 will be explained with reference to FIG. 21. FIG. 21 is a block diagram showing a schematic configuration of the image display apparatus 304 shown in FIG. 20. As shown in FIG. 21, the image display apparatus 304 has an input unit 320, a display unit 330, a storage unit 340, and a control unit 350.

The input unit 320 has an information input unit 321 inputting selection information selecting a desired image from images sequentially displayed on the display unit 330. The input unit 320 is realized by such a pointing device such as a keyboard or a mouse to feed an operation information of the image display apparatus 304 and instruction information of a process performed by the image display apparatus 304 to the control unit 350.

The display unit 320 is realized by a CRT display, a liquid crystal display, or the like to display and output instruction information of the input unit 320, an instruction result, or the like. The display unit 330 sequentially displays respective images in an image group imaged by the capsule endoscope 303 in a predetermined pseudo moving image area in an imaging order of the images as the pseudo moving images. The display unit 330 displays and outputs a reduced image of a selected image according to selection information of the selection information input unit 321 in a predetermined reduced image display area.

The control unit 340 is realized by, for example, a hard disk device, where various images are saved and particularly an image group PG1 imaged by the capsule endoscope 303 and a reduced image group PG2 produced in the control unit 350 are respectively stored in folders F1 and F2.

The control unit 350 controls respective processes or operations of the input unit 320, the display unit 330, and the storage unit 340. The control unit 350 includes a selected image extracting unit 352, and an image display controller 355 having a reduced image generator 353 and a timer 354. The selected image extracting unit 352 extracts a selected image from the folder F1 based on selection information inputted from the selection information input unit 321. The reduced image generator 353 performs a thinning process of pixels, a determination process based on linear interpolation of values of pixels to be displayed, or the like to produce a reduced image with regard to an image extracted by the selected image extracting unit 352. The reduced image generator 353 produces an image smaller than an image displayed in the pseudo moving image display area of the display unit 330 as a reduced image to the image extracted by the selected image extracting unit 352. The timer 354 has a function of measuring time. The image display controller 355 controls an image display process in the display unit 330. The image display controller 355 causes the display unit 330 to display a reduced image produced by the reduced image generator 353 in the reduced image display area. In other words, the image display controller 355 causes the display unit 330 to display an image extracted by the selected image extracting unit 352 in the reduced image display area as an image smaller than the image displayed in the pseudo moving image display area.

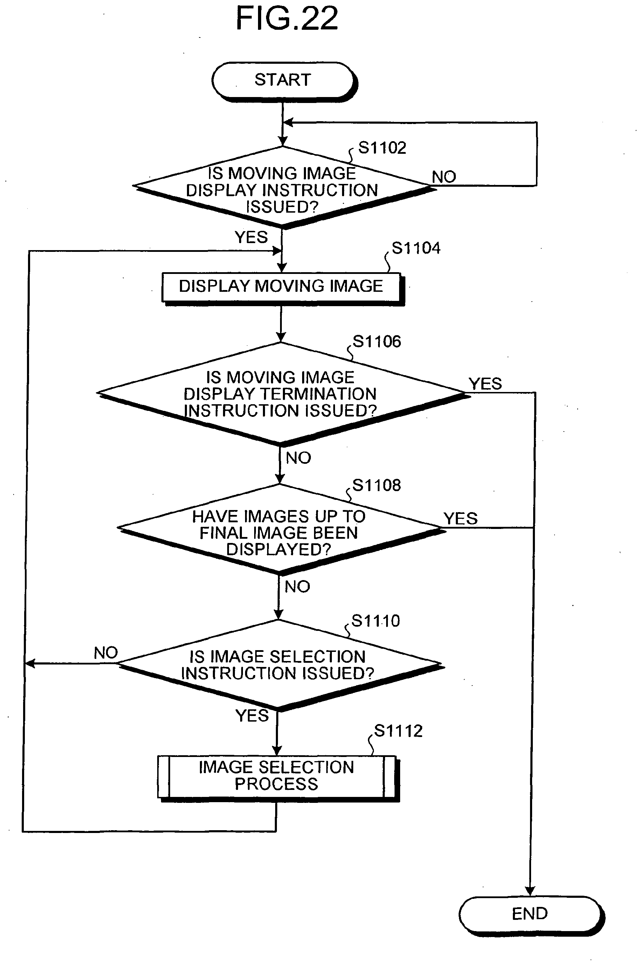

Next, an image display process procedure performed by the control unit 350 will be explained with reference to FIG. 22. In FIG. 22, the control unit 350 determines whether instruction information instructing pseudo moving image display is issued from the input unit 320 (step S1102). The control unit 350 repeats determination at step S1102 until instruction information instructing pseudo moving image display is issued, and when instruction information instructing pseudo moving image display is issued (step S1102, Yes), the image display controller 355 causes the display unit 330 to perform pseudo moving image display that sequentially displays respective images of the image group PG1 stored in the folder F1 of the storage unit 340 in the predetermined pseudo moving image display area according to an imaging order (step S1104).

The control unit 350 determines whether instruction information instructing termination of pseudo moving image display from the input unit 320 is issued (step S1106), and when the instruction information is issued (step S1106, Yes), the control unit 350 terminates the pseudo moving image display. On the other hand, the control unit 350 determines that instruction information indicating termination of the pseudo moving image display from the input unit is not issued (step S1106, No), the image display controller 355 determines whether the display unit 330 has displayed images up to the final image in the image group PG1 (step S1108). When the image display controller 355 determines that the display unit 330 has displayed images up to the final image in the image group PG1 (step S1108, Yes), the control unit 350 terminates the pseudo moving image display.

On the other hand, when the control unit 350 determines that the display unit 330 has not displayed images up to the final image in the image group PG1 (step S1108, No), it determines whether an image selection instruction has been issued based on presence/absence of selection information from the selection information input unit 321 (step S1110). When the control unit 350 determines that image selection instruction is not issued (step S1110, No), the control unit 350 proceeds to step S1104 to continue the pseudo moving image display.

On the other hand, when the control unit 350 determines that selection information is inputted from the selection information input unit 321 and an image selection instruction has been issued (step S1110, Yes), the control unit 350 reduces a selected image based on the selection information to perform an image selection process that causes the display unit 330 to display a reduced image in the predetermined reduced image display area (step S1112). After the control unit 350 performs the image selection process, it proceeds to step S1104 to perform pseudo moving image display.

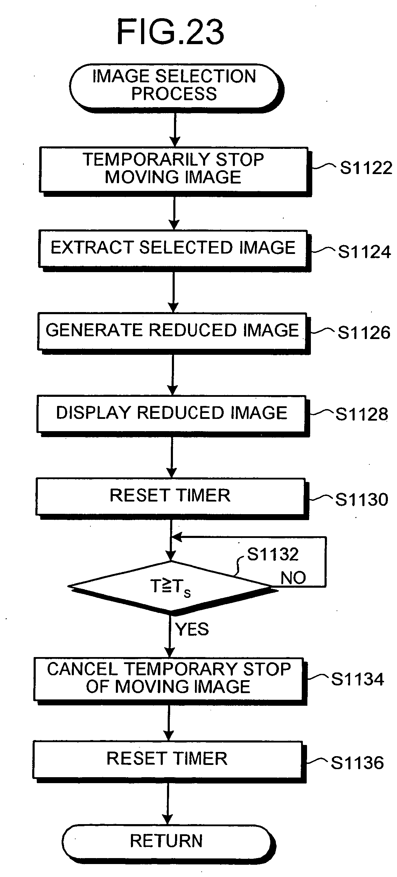

Next, an image selection process shown in FIG. 22 will be explained. FIG. 23 is a flowchart showing a process procedure of the image selection process shown in FIG. 22. As shown in FIG. 23, the image display controller 355 causes the display unit 330 to temporarily stop sequential display of the pseudo moving image displayed in the pseudo moving image area (step S1122) and display a stopped image in the pseudo moving image area. The selected image extracting unit 352 extracts a selected image from the folder F1 in the storage unit 340 based on selection information inputted from the selection information input unit 321 (step S1124). The reduced image generator 353 generates a reduced image to the image extracted by the selected image extracting unit 352 (step S1126). The image display controller 355 causes the display unit 330 to display a reduced image produced by the reduced image generator 353 in a predetermined reduced image display area (step S1128).

Thereafter, the image display controller 355 starts timing of the timer 354 (step S1130). The image display controller 355 determines whether a timed value T of the timer 354 is equal to or more than a predetermined waiting time Ts (step S1132) and it repeats determination at step S1132 until the timed value T reaches the waiting time Ts or more. When the image display controller 355 determines that the timed value T is equal to or more than the predetermined waiting time Ts (step S1132, Yes), it cancels the temporary stopping of sequential display of the pseudo moving image display (step S1134). That is, the image display controller 355 instructs the display unit 330 to restart sequential display of the pseudo moving image display. Thereafter, the image display controller 355 resets the timed value of the timer 354 to 0 and stops it (step S1136).

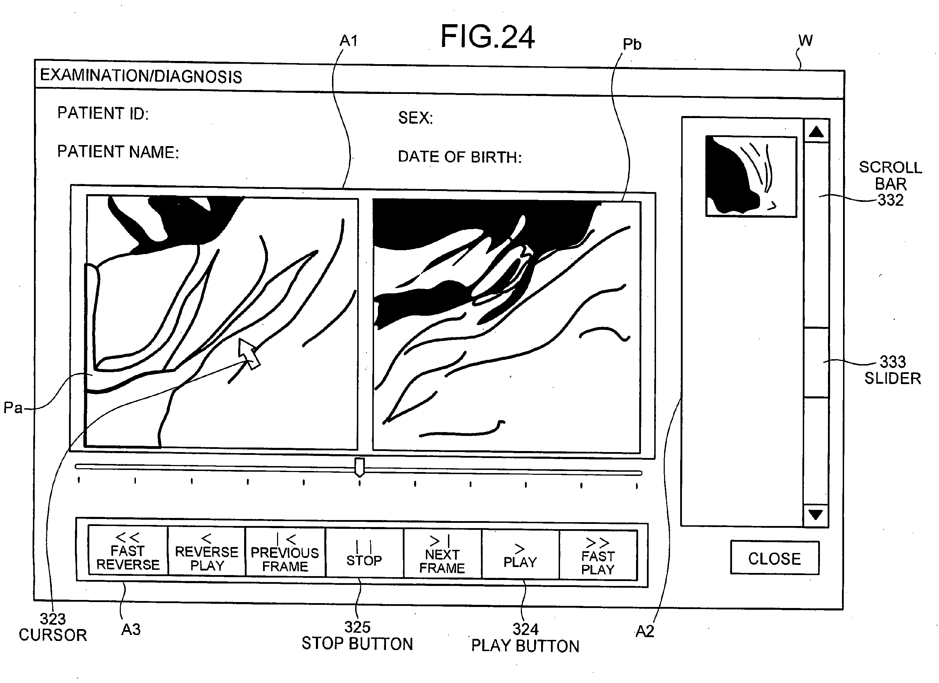

A specific operation and process will be explained with reference to FIG. 24 to FIG. 26. FIG. 24 to FIG. 26 are diagrams showing one example of a display screen of the display unit 330, where a window W is displayed on a display screen of the display unit 330. The window W roughly includes a pseudo moving image display area A1, a reduced image display area A2, and a moving image display control key area A3, where the reduced image display area A2 is provided on a right side area of the pseudo moving image display area A1 and the moving image display control key area A3 is provided below the pseudo moving image display area A1. Images in the folder F1 stored in the storage unit 340 are sequentially displayed in the pseudo moving image display area A1 in an imaging order of the images, a reduced image produced by the reduced image generator 353 is displayed in the reduced image display area A2, and a button image showing various display control processes of pseudo moving images such as a play button 324 and a STOP button 325 is displayed in the moving image display control key area A3. When a reduced image can not be displayed on one screen, a scroll bar 332 is displayed in the reduced image display area A2. A user moves a slider 333 on a screen vertically to change a display content in the reduced image display area A2 vertically by moving the mouse of the input unit 320 to move a cursor 323 moved on the slider 333 vertically.

Instruction information for instructing start of pseudo moving image display is inputted in the control unit 350 by moving the cursor 323 shown in FIG. 24 on the play button 324 to click a left button on the mouse according to an operation of the mouse of the input unit 320 or the like. As a result, as shown in FIG. 24, an image Pa is displayed in a left side area of the pseudo moving image display area A1, and an image Pb imaged by the capsule endoscope 303 is displayed next to the image Pa in a right side area of the pseudo moving image display area A1. Thereafter, an image taken by the capsule endoscope 303 is displayed next to the image Pb in the left side area of the pseudo moving image display area A1, and an image taken by the capsule endoscope 303 is displayed next to the image displayed on the left side in the right side area of the pseudo moving image display area A1.

For example, when the image Pa is selected as a desired image, a user moves the mouse of the input unit 320 to move the cursor 323 on the image Pa to double-click the left button on the mouse so that selection information selecting the image Pa is inputted into the control unit 350 from the selected image input unit 21 of the input unit 320.

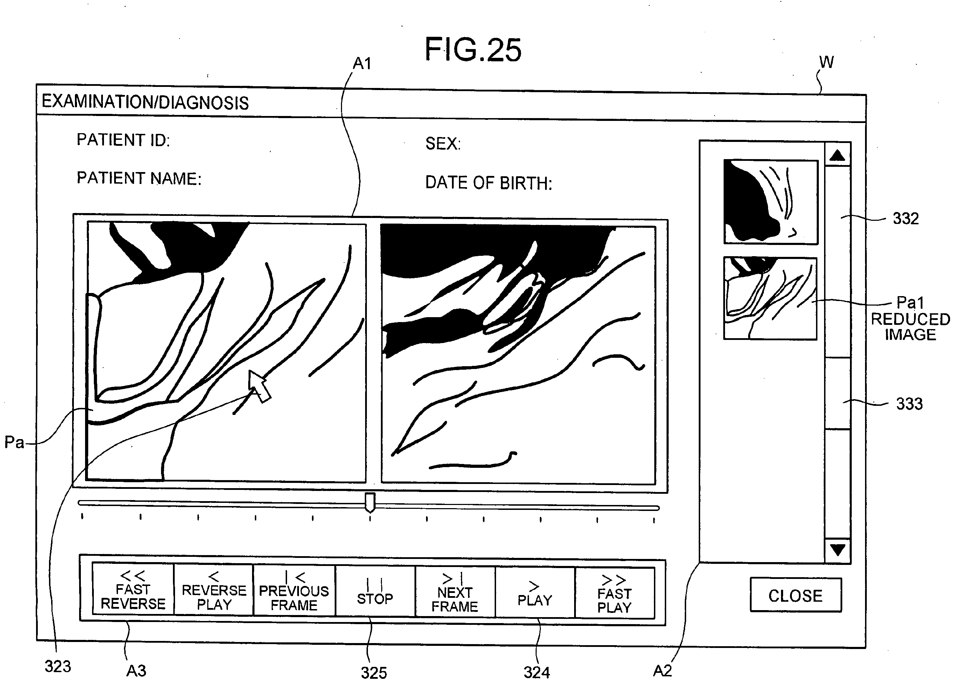

When the selected information is inputted into the control unit 350, the image display controller 355 first causes the display unit 330 to temporarily stop sequential display of a pseudo moving image. As a result, display of the images Pa and Pb continues in the pseudo moving image display area A1. The selected image extracting unit 352 extracts the image Pa selected from the folder F1 in the storage unit 340 based on the selection information and the reduced image generator 353 produces a reduced image Pa1 to the extracted image Pa. As shown in FIG. 25, the image display controller 355 causes the display unit 330 to display the reduced image Pa1 produced by the reduced image generator 353 in the reduced image display area A2. As a result, the reduced image Pa1 to the selected image Pa is displayed in the reduced image display area A2.

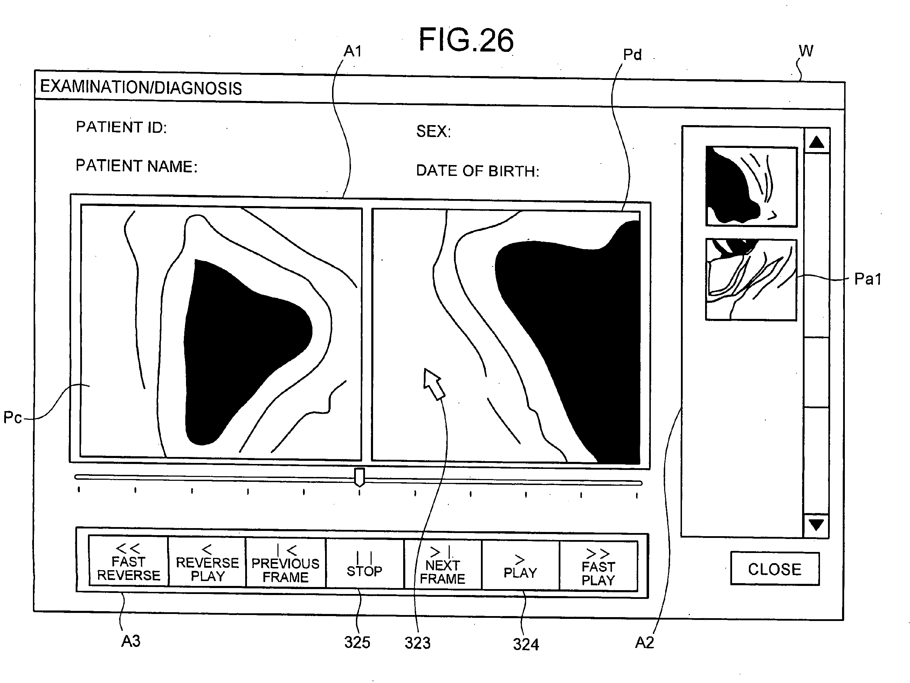

Thereafter, when timing of the timer 354 starts and the timed value T of the timer 354 reaches the waiting time Ts or more, sequential display of images is restarted in the pseudo moving image display area of the display unit 330 according to display control of the image display controller 355. As a result, as shown in FIG. 26, images Pc and Pd taken by the capsule endoscope .303 are displayed next to the images Pa and Pb in the pseudo moving image display area A1. Thus, after selection of the image Pa, sequential display of images in the pseudo moving image display area A1 is stopped during a period from the display of the reduced image Pa1 and until the elapse of the waiting time Ts, and the images Pa and Pb are continuously displayed.

According to the image display apparatus 304 according to the third embodiment, by displaying an image selected by a user from images sequentially displayed in the predetermined pseudo moving image display area in the predetermined selected image display area that is a display area different from the pseudo moving image display area, rapid confirmation of a selected image performed by the user is made possible. Since the image display apparatus 304 reduces and displays images selected by the user from images sequentially displayed in the predetermined pseudo moving image display area in the predetermined image display area which is another display area in an imaging order to the images, the user can recognize the imaging order of the selected images reliably.

In the third embodiment, after the selection information is inputted, sequential display of the pseudo moving images is stopped during a period from the display of the reduced image and until the elapse of a predetermined waiting time. In other word, moving images in the pseudo moving image display area A1 is stopped after the user selects a desired image during a period from the display of a reduced image of the selected image in the reduced image display area A2 of the window W and until the elapse of the predetermined waiting time Ts. Further, in the third embodiment, thus, when the user selects an image, moving images of the pseudo moving images are stopped so that user's omission of an image in the pseudo moving images can be prevented. In the third embodiment, since moving images in the pseudo moving image display are stopped during a period from the display of a reduced image and until the elapse of the predetermined waiting time Ts, even while the user is viewing the reduced image display area, sufficient time can be secured for moving user's view to the pseudo moving image display area, so that user's omission of pseudo moving images can be prevented. As a result, the user's burden at a selecting time of an image required for diagnosis can be reduced. Accordingly, by using the image display apparatus 304 according to the third embodiment, improvement of diagnosis using an endoscope image performed by the user can be achieved.

The user inputs selection information to a desired image by double-clicking the left button on the mouse in a state that the cursor has been directly moved on a desired image. Therefore, it is unnecessary for the user to move the cursor 323 in the moving image display control key area A3. Accordingly, in the third embodiment, since mouse movement at the image selection time can be minimized, user's burden at the image selection time can be reduced.

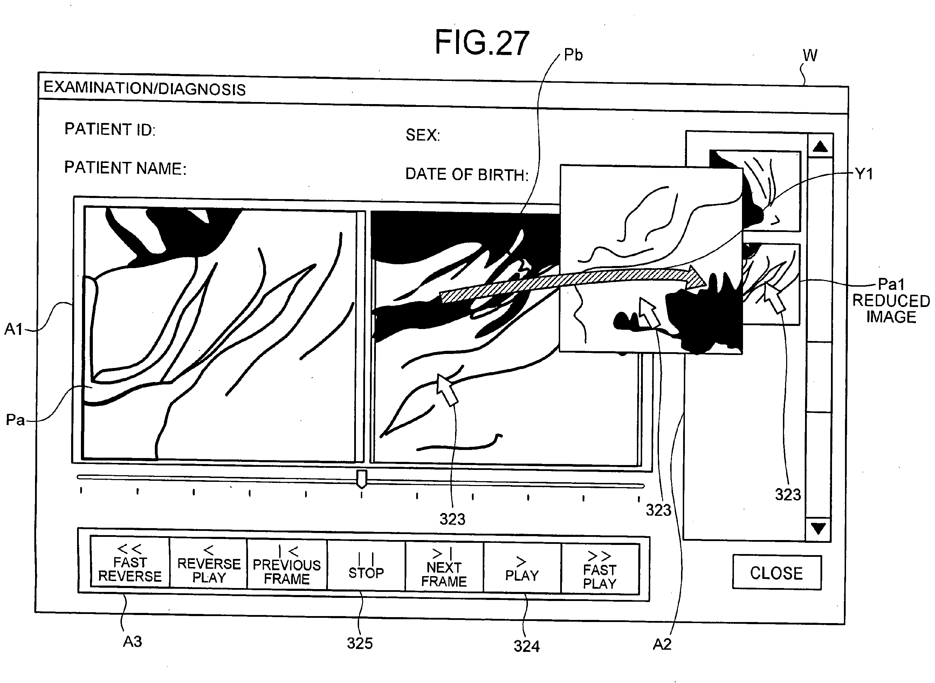

In the third embodiment, the case that selection information is inputted by double clicking of the left button on the mouse has been explained with reference to FIG. 24 to FIG. 26, however, the present invention is not limited to this case. The selection information can be inputted by single clicking or clicking of a right button on the mouse. The selection information can be inputted by performing a so-called drag and drop. For example, a case that the image Pb shown in FIG. 27 is selected will be explained. A user moves the mouse of the input unit 320 to move the cursor 323 on the image Pb, presses the button on the mouse in a state that the cursor 323 is superimposed on the image Pb, moves the mouse in a direction of arrow Y1 as it is, moves the cursor 323 to the reduced image display area A2, and releases the button on the mouse in the reduced image display area A2. In the third embodiment, selection information for selecting a desired image can be inputted according to such a drag and drop operation.

### Fourth Embodiment

Next, a fourth embodiment will be explained. In the third embodiment, after the predetermined waiting time elapse from the display of a reduced image, sequential display of stopped images is restarted.. In the fourth embodiment, however, after instruction information instructing restart of sequential display of images is inputted, sequential display of stopped images is restarted.

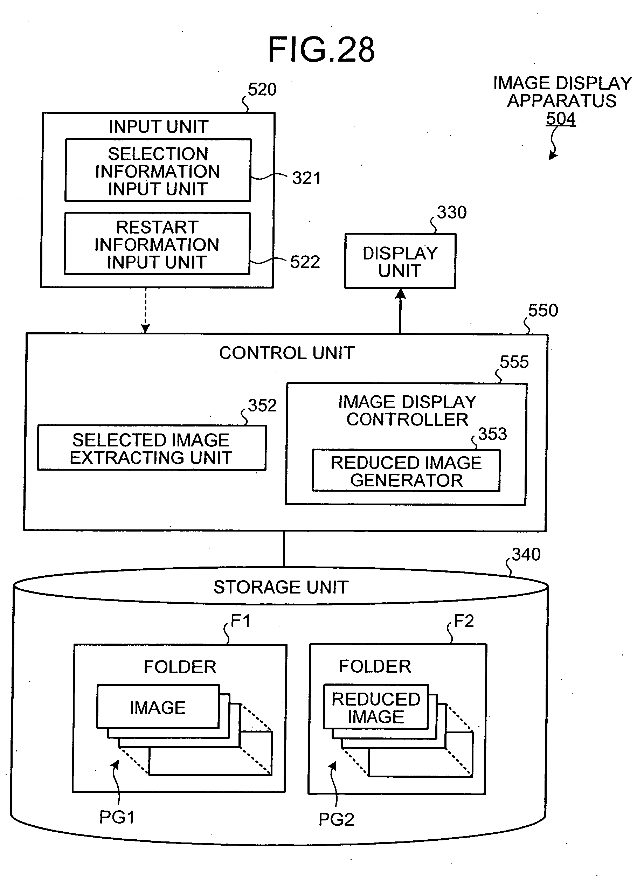

FIG. 28 is a block diagram showing a schematic configuration of an image display apparatus according to the fourth embodiment. As shown in FIG. 28, an image display apparatus 504 according to the fourth embodiment includes an input unit 520 further including a restart information input unit 522 instead of the input unit 320, which is different from the image display apparatus 304 according to the third embodiment. A control unit 550 includes an image display controller 555 having a configuration where the timer has been removed instead of the image display controller 355. The input unit 520 has a function equivalent to that of the input unit 320 in the third embodiment, and the restart information input unit 522 inputs restart information instructing restart of sequential display of images in the temporarily stopped pseudo moving images. The control unit 550 has a function equivalent to that of the control unit 350 in the third embodiment. The image display controller 555 has a function equivalent to that of the image display controller 355 in the third embodiment and it restarts the display unit 330 to perform sequential display of images of the pseudo moving images based on the restart information inputted from the restart information input unit 522 in the input unit 520. Other configurations are the same as those of the third embodiment, and like constituent elements are denoted with like reference letters or numerals.

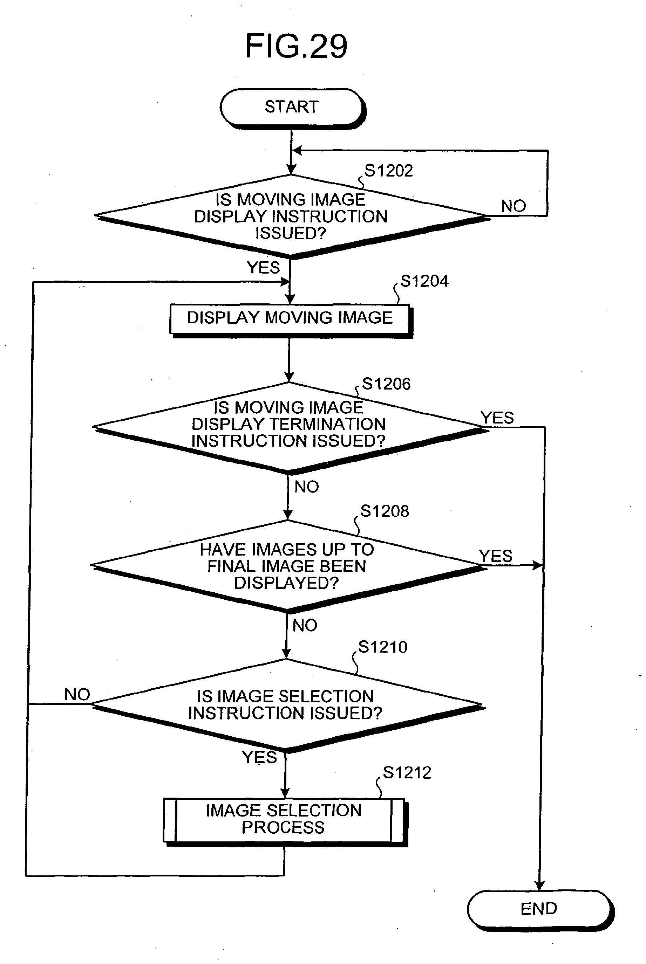

Next, an image display process procedure performed by the control unit 550 will be explained with reference to FIG. 29. The control unit 550 first determines whether instruction information instructing pseudo moving image display is issued from the input unit 520 (step S1202) like the third embodiment, and when the control unit 550 determines that the instruction information has been issued (step S1202, Yes), it causes the display unit 330 to perform pseudo moving image display of the image group PG1 (step S1204). Next, the control unit 550 determines whether instruction information instructing termination of the pseudo moving image display has been issued from the input unit 520 (step S1206) like the third embodiment, and when the control unit 550 determines that the instruction information has been issued (step S1206, Yes), it causes the display unit 330 to terminate the pseudo moving image display. On the other hand, the control unit 550 determines that instruction information indicating termination of the pseudo moving image display is not issued (step S1206, No), the control unit 550 determines whether images up to the final image of the image group PG1 as the pseudo moving images have been displayed (step S1208) like the third embodiment. When the control unit 550 determines that images up to the final image have been displayed (step 1208, Yes), it causes the display unit 330 to terminate the pseudo moving image display. On the other hand, the control unit 550 determines that images up to the final image have not been displayed (step 1208, No), it determines whether an image selection instruction has been issued based on presence/absence of selection information from the selection information input unit 321 (step S1210). When the control unit 550 determines that the image selection instruction has not been issued (step S1210, No), it proceeds to step S1204 to cause the display unit 330 to continue the pseudo moving image display. As explained in FIG. 24 to FIG. 26, the selection information can be inputted by double clicking of the left button on the mouse of the input unit 520, or inputting can be performed according to a drag and drop operation, as explained in FIG. 27.

On the other hand, when the control unit 550 determines that the image selection instruction has been issued (step S1210, Yes), it produces a reduced image to a selected image to perform an image selection process to cause the display unit 330 to display the reduced image in the predetermined reduced display area (step S1212). After the control unit 555 performs the image selection process, it proceeds to step S1204 to perform the pseudo moving image display.

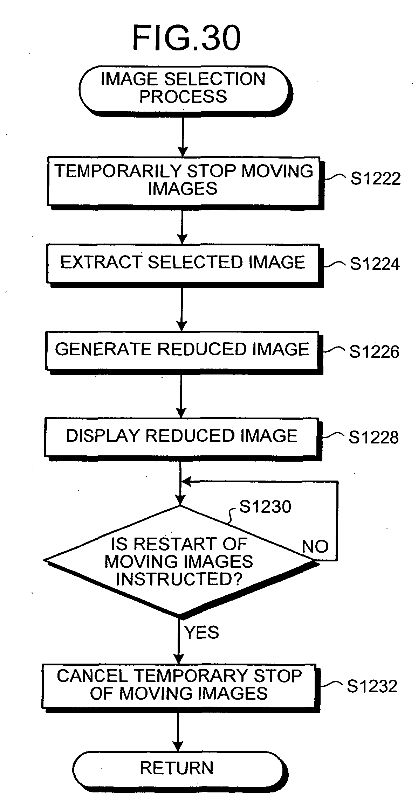

Next, an image selection process shown in FIG. 29 will be explained. FIG. 30 is a flowchart showing a process procedure of the image selection process shown in FIG. 29. As shown in FIG. 30, the image display controller 555 causes the display unit 330 to temporarily stop sequential display of pseudo moving image display like the third embodiment (step S1222), and the selected image extracting unit 352 extracts an image selected from the folder F1 of the storage unit 340 based on selection information inputted from the selection information input unit 321 (step S1224). The reduced image generator 353 generates a reduced image to the image extracted by the selected image extracting unit 352 (step S1226), and the image display controller 555 causes the display unit 330 to display the reduced image produced by the reduced image generator 353 in a predetermined image display area A2 (step S1228).

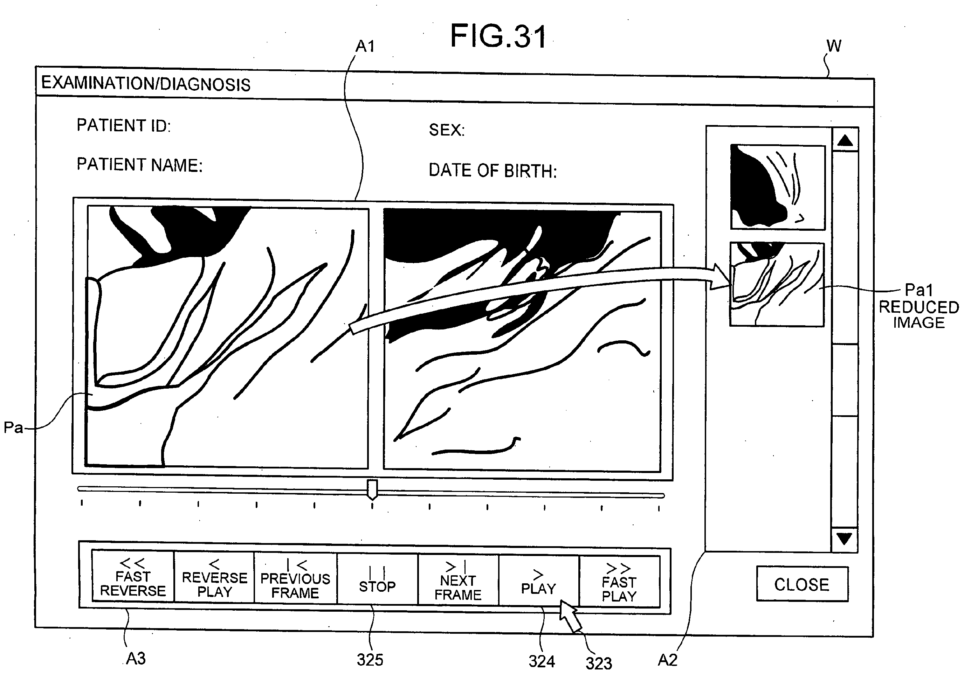

Thereafter, the image display controller 555 determines whether restart instruction of sequential display of the pseudo moving image display has been issued based on presence/absence of restart information indicating restart of the sequential display of the pseudo moving image display from the restart information input unit 522 in the input unit 520 (step S1230). For example, as shown in FIG. 31, the restart information is inputted by moving the cursor 323 on the restart button 324 to click the left button on the mouse.

The image display controller 555 repeats determination at step S1230 until the restart instruction is issued. When the image display controller 555 determines that the restart instruction has been issued (step S1230, Yes), it causes the display unit 330 to cancel the temporary stop of sequential display of the pseudo moving images (step S1232). The image display controller 555 proceeds to step 204 in FIG. 29, where the display unit 330 restarts sequential display of the pseudo moving images.

Thus, in the fourth embodiment, since sequential display of images is stopped until instruction information instructing restart of sequential display of images is inputted, an effect similar to that in the third embodiment can be achieved, and since a user oneself can instruct restart of sequential display of pseudo moving images, omission of the pseudo moving images can be reliably prevented.

In the third and fourth embodiments, the case that two images are displayed in the pseudo moving image display area A1 has been explained with reference to FIG. 24 to FIG. 27 and FIG. 31, but one image can be displayed in the pseudo moving image display area A1, four images can be displayed thereon, where the number-of-display screen of images is not limited, of course. The number of images to be displayed in the pseudo moving image display area A1 can be changed based on instruction information of the input unit 320 or 520.

In the third and fourth embodiments, the image display apparatuses 304 and 504 that perform restart of sequential display of pseudo moving image according to the predetermined waiting time elapse or presence/absence of the restart information has been explained, however, the present invention is not limited to these the image display apparatuses. An image display apparatus where a condition for restart of sequential display of pseudo moving images can be switched to either one of the predetermined waiting time elapse and the presence/absence of the restart information can be used. In this case, either mode of the predetermined waiting time elapse and the presence/absence of the restart information is selected as the condition for restart of sequential display of pseudo moving images prior to inputting start of the pseudo moving image display. When default of restart condition of sequential display of pseudo moving images is set as the predetermined waiting time elapse and presence/absence of restart information is set as the restart condition, the presence/absence of restart information can be designated as the restart condition of sequential display of pseudo moving images.

By moving the cursor 323 on the temporary stop button 325 shown in FIG. 24 to FIG. 27 and FIG. 31 to click the button, temporary stop of sequentially display of pseudo moving images is instructed and by clicking the temporary stop button 325 again, the sequentially display of pseudo moving images is restarted.

An image display apparatus according to any one of the first to the fourth embodiments (for example, the image display apparatuses 4, 21, 304, and 504) can be realized by executing a preliminarily prepared program utilizing a computer system such as a personal computer or a workstation. A computer system executing an image display program having a function similar to that of the image display apparatus according to any one of the first to fourth embodiments will be explained below.

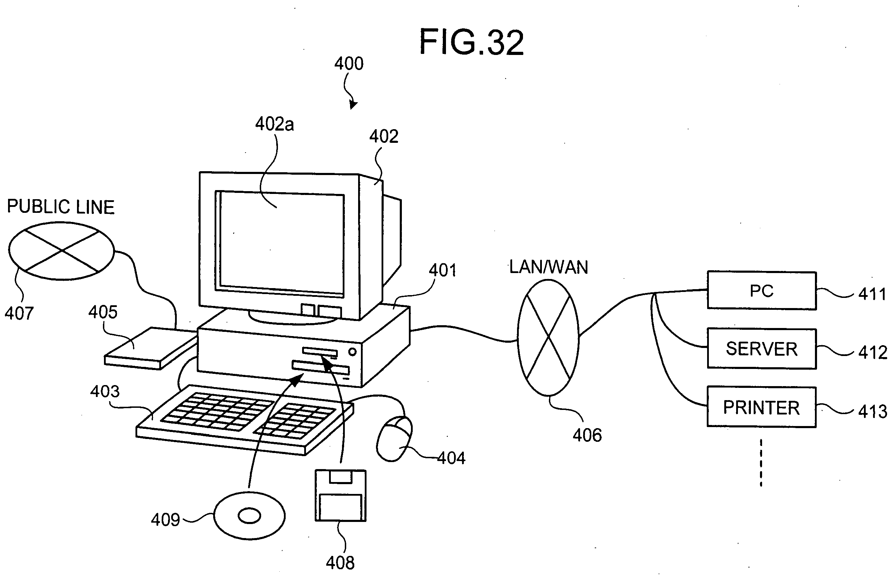

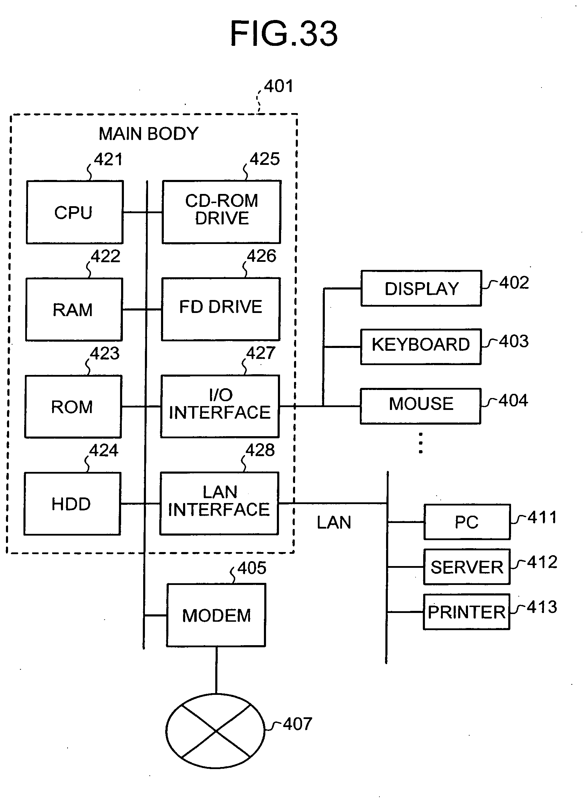

FIG. 32 is a configuration diagram showing a configuration of a computer system using any one of the first to fourth embodiments, and FIG. 33 is a block diagram showing a configuration of a main body in the computer system. As shown in FIG. 32, a computer system 400 according to the embodiment includes a main body 401, a display 402 for displaying information such as an image on a display screen 402a according to an instruction from the main body 401, a keyboard 403 for inputting various information into the computer system 400, and a mouse 404 for designating an arbitrary position on a display screen 402a of the display 402.

As shown in FIG. 33, the main body 401 in the computer system 400 includes a CPU 421, a RAM 422, a ROM 423, a hard disk drive (HDD) 424, a CD-ROM drive 425 that accommodates a CD-ROM 409, an FD drive 426 that accommodates a flexible disk (FD) 408, an I/O interface 427 connecting the display 402, the keyboard 403, and the mouse 404, and a LAN interface 428 connected to a local area network or a wide area network (LAN/WAN) 406.

The computer system 400 is connected with a modem 405 for connection to a public line 407 such as the Internet and is connected with another computer system (PC) 411, a server 412, a printer 413, and the like via the LAN interface 428 and the LAN/WAN 406.

The computer system 400 realizes an image display apparatus by reading and executing an image display program recorded in a predetermined recording medium. The predetermined recording medium includes any recording medium recording an image display program that can be read by the computer system 400, including "portable physical medium" such as a flexible disk (FD) 408, the CD-ROM 409, an MO disk, a DVD disk, a magnetic optical disk, or an IC card, and also "fixed physical medium" such as the hard disk drive (HDD) 424, the RAM 422, or the ROM 423 provided outside or inside the computer system 400, and "communication medium" holding a program for a short period during transmission of a program, such as the public line 407 connected via the modem 405 or the LAN/WAN 406 connected with another computer system 411 and another server 412.

That is, the image display program is recorded on a recording medium such as the "potable physical medium", "fixed physical medium", and "communication medium" in a computer-readable manner, and the computer system 400 can realize an image display apparatus and an image display method by reading the image display program from such a recording medium and executing the same. The image display program is not limited to the one executed by the computer system 400, but similarly applied to a case that another computer system 411 or the server 412 executes the image display program, or to a case that these computers execute the image display program in cooperation with each other.

On the other hand, by combining the image display apparatus according to the first or the second embodiment (for example, the image display apparatus 4 or the image display apparatus 21) and the image display apparatus according to the third or the fourth embodiment (for example, the image display apparatus 304 or the image display apparatus 504), an image display apparatus that can achieve both the operational effects of the first or the second embodiment and the operational effects of the third or the fourth embodiment can be realized. Specifically, such an image display apparatus can be realized, for example, by adding a function of the selection information input unit 321 (and further a function of the restart information input unit 522) to the input unit 10 of the image display apparatus 4 according to the first embodiment, storing the folders F1 and F2 in the storage unit 14, and further adding a function of the selected image extracting unit 352 and a function of the image display controller 355 (or a function of the image display controller 555) to the control unit 15. Such an image display apparatus can be realized, for example, by adding a function of the selection information input unit 321 (and further a function of the restart information input unit 522) to the input unit 10 of the image display apparatus 21 according to the second embodiment, further storing the folders F1 and F2 in the storage unit 14, and further adding a function of the selected image extracting unit 352 and a function of the image display controller 355 (or a function of the image display controller 555) to the control unit 22.

### INDUSTRIAL APPLICABILITY

As described above, the image display apparatus, the image display method, and the image display program according to the present invention are useful in an apparatus that displays a series of images taken by sequentially imaging a desired subject in time series, and are particularly suitable for an image display apparatus that can easily observe a series of images of inside of the body of a subject sequentially taken by a capsule endoscope.

## Claims

1. An image display apparatus (4, 21) including a display unit (11) that displays a series of images taken by imaging a desired subject (1) in time series,
**characterized in that** the image display apparatus (4) includes:
a control unit (15) that controls the display unit (11) to display a time scale (140) indicating an imaging period of the series of images, to display a specific image (SP1-5) specifying a desired image selected from the series of images, wherein the control unit (15) is adapted to acquire imaging date and time information of the desired image (SP1-5), to calculate a time elapsed from the imaging start date and time until the desired image (SP1-5) is taken based on the imaging date and time information and the imaging start date and time information, to determine a position on the time scale (140) associated with the desired image (SP1-5), and to control the display unit (11) to display a line connecting the specific image (SP1-5) and the position on the time scale (140) corresponding to an imaging time of the desired image (SP1-5).

2. The image display apparatus according to claim 1, wherein the control unit (15) controls the display unit (11) to display at most a predetermined number of desired images (SP1-5) specifying the desired images on the same screen and to display the index for each of the desired images (SP1-5) displayed on the same screen.

3. The image display apparatus according to claim 2, comprising an image designating (10) unit that designates a desired image (SP1-5) of a displayed object of the index from the desired images (SP1-5) displayed on the same screen, wherein the control unit (15) controls the display unit (10) to display the index for each of the desired images (SP1-5) designated by the image designating unit (10).

4. The image display apparatus according to claim 1, wherein the desired image (SP1-5) is a thumbnail corresponding to the designated image.

5. The image display apparatus according to one of claims 1 to 4, wherein the subject is an examinee subjected to an internal-body examination, and the control unit (15) detects an imaging period of external body images taken by imaging an external body of the examinee, and controls the display unit to display an external body image period mark (142) indicating an imaging period of the external body images of the imaging period of the series of images.

6. The image display apparatus according to one of claims 1 to 4, wherein the control unit (15) detects an imaging period where identical images continue in the series of images, and the control unit controls the display unit to display an identical image period mark (143, 144) indicating the imaging period of the identical images of the imaging period of the series of images.

7. An image display program that displays a series of images taken by imaging a desired subject (1) in time series and displays a time scale (140) indicating an imaging period of the series of images, and that causes a computer to execute:
an information extracting procedure of extracting information corresponding to an imaging time of a desired image (101) selected from the series of images; **characterized by** displaying a specific image (SP1-5) specifying a desired image selected from the series of images, acquiring imaging date and time information of the desired image (SP1-5), to calculate a time elapsed from the imaging start date and time until the desired image (SP1-5) is taken based on the imaging date and time information and the imaging start date and time information, to determine a position on the time scale (140) associated with the desired image (SP1-5) and displaying a line connecting the specific image (SP1-5) and a position on the time scale (140) corresponding to the imaging time of the desired image (SP1-5).

8. The image display apparatus of any preceding claim, wherein
the display apparatus further comprises an input unit (10); and
the desired images are selected when a user performs one of a key operation or a click operation of selecting an image displayed on a main-image display area (100) displayed on the display unit (11) using the input unit (10).

9. The image display apparatus of claim 8, wherein a plurality of desired images (SP1-5) are selected by the user performing the one of the key operation or the click operation.

## Patentansprüche

1. Bildanzeigevorrichtung (4, 21), die eine Anzeigeeinheit (11) umfasst, die eine Reihe von Bildern anzeigt, die durch Abbilden eines gewünschten Subjekts (1) in Zeitreihen aufgenommen wurden,
**dadurch gekennzeichnet, dass** die Bildanzeigevorrichtung (4) umfasst:
eine Steuerungseinheit (15), die die Anzeigeeinheit (11) steuert, um eine Zeitskala (140) anzuzeigen, die einen Abbildungszeitraum der Bilderreihen darstellt, um ein spezifisches Bild (SP1-5) anzuzeigen, das ein gewünschtes Bild spezifiziert, das aus der Bilderreihe ausgewählt wurde, wobei die Steuerungseinheit (15) dazu angepasst ist, eine Abbildungsdatum- und -zeitinformation des gewünschten Bilds (SP1-5) zu erfassen, um eine Zeit, die von dem Datum und der Zeit des Abbildungsstarts bis zur Aufnahme des gewünschten Bilds (SP1-5) vergangen ist, basierend auf dem Abbildungsdatum und der Zeitinformation und der Abbildungsstartdatums- und - zeitinformation zu berechnen, um eine Position auf der Zeitskala (140) zu bestimmen, die mit dem gewünschten Bild (SP1-5) verknüpft ist, und um die Anzeigeeinheit (11) zu steuern, um eine Linie anzuzeigen, die das spezifische Bild (SP1-5) und die Position auf der Zeitskala (140), die einer Abbildungszeit des gewünschten Bilds (SP1-5) entspricht, verbindet.

2. Bildanzeigevorrichtung nach Anspruch 1, wobei die Steuerungseinheit (15) die Anzeigeeinheit (11) steuert, um höchstens eine vorbestimmte Anzahl an gewünschten Bildern (SP1-5), die die gewünschten Bilder spezifizieren, auf demselben Bildschirm anzuzeigen und um den Index für jedes der auf demselben Bildschirm angezeigten gewünschten Bilder (SP1-5) anzuzeigen.

3. Bildanzeigevorrichtung nach Anspruch 2, die eine Bildkennzeichnungseinheit (10) umfasst, die ein gewünschtes Bild (SP1-5) eines angezeigten Objekts des Index der gewünschten Bilder (SP1-5), die auf demselben Bildschirm angezeigt werden, kennzeichnet, wobei die Steuerungseinheit (15) die Anzeigeeinheit (10) steuert, um den Index für jedes der von der Bildkennzeichnungseinheit (10) gekennzeichneten gewünschten Bilder (SP1-5) anzuzeigen.

4. Bildanzeigevorrichtung nach Anspruch 1, wobei das gewünschte Bild (SP1-5) ein Miniaturbild ist, das dem gekennzeichneten Bild entspricht.

5. Bildanzeigevorrichtung nach einem der Ansprüche 1 bis 4, wobei das Subjekt eine zu untersuchende Person ist, die einer Untersuchung des Körperinneren unterzogen wird, und die Steuerungseinheit (15) einen Abbildungszeitraum von Bildern des äußeren Körpers erfasst, die durch Abbildung eines äußeren Körpers der zu untersuchenden Person aufgenommen wurden, und die Anzeigeeinheit steuert, um eine Zeitraummarkierung (142) für ein äußeres Körperbild anzuzeigen, die einen Abbildungszeitraum der äußeren Körperbilder des Abbildungszeitraums der Bilderreihen darstellt.

6. Bildanzeigevorrichtung nach einem der Ansprüche 1 bis 4, wobei die Steuerungseinheit (15) einen Abbildungszeitraum erfasst, in dem identische Bilder in den Bilderreihen folgen und die Steuerungseinheit die Anzeigeeinheit steuert, um eine Zeitraummarkierung (143, 144) für ein identisches Bild anzuzeigen, die den Abbildungszeitraum der identischen Bilder des Abbildungszeitraums der Bilderreihen darstellt.

7. Bildanzeigeprogramm, das eine Reihe von Bildern anzeigt, die durch Abbilden eines gewünschten Subjekts (1) in Zeitreihen aufgenommen wurden, und eine Zeitskala (140) anzeigt, die einen Abbildungszeitraum der Bilderreihen darstellt, und das bewirkt, dass ein Computer ausführt:
ein Informationsextrahierverfahren zum Extrahieren einer Information, die einer Abbildungszeit eines aus den Bilderreihen ausgewählten gewünschten Bilds (101) entspricht; **gekennzeichnet durch** Anzeigen eines spezifischen Bilds (SP1-5), das ein aus den Bilderreihen ausgewähltes gewünschtes Bild spezifiziert, Erfassen einer Abbildungsdatums- und -zeitinformation des gewünschten Bilds (SP1-5), um eine Zeit, die von dem Datum und der Zeit des Abbildungsstarts bis zur Aufnahme des gewünschten Bilds (SP1-5) vergangen ist, basierend auf dem Abbildungsdatum und der Zeitinformation und der Abbildungsdatums- und -zeitinformation zu berechnen, um eine Position auf der Zeitskala (140) zu bestimmen, die mit dem gewünschten Bild (SP1-5) verknüpft ist, und Anzeigen einer Linie, die das spezifische Bild (SP1-5) und eine Position auf der Zeitskala (140), die der Abbildungszeit des gewünschten Bilds (SP1-5) entspricht, verbindet.

8. Abbildungsvorrichtung nach einem vorhergehenden Anspruch, wobei die Anzeigevorrichtung ferner eine Eingabeeinheit (10) umfasst; und die gewünschten Bilder ausgewählt werden, wenn ein Benutzer eine von einer Tastaturbedienung oder einer Mausklickbedienung zum Auswählen eines auf einer Hauptbildanzeigefläche (100) angezeigten Bilds, das auf der Anzeigeeinheit (11) angezeigt wird, mithilfe der Eingabeeinheit (10) durchführt.

9. Bildanzeigevorrichtung nach Anspruch 8, wobei eine Mehrzahl von gewünschten Bildern (SP1-5) durch den Benutzer ausgewählt werden, der die eine von der Tastaturbedienung oder der Mausklickbedienung durchführt.

## Revendications

1. Appareil d'affichage d'image (4, 21) comprenant une unité d'affichage (11) qui affiche une série d'images prises par imagerie d'un sujet souhaité (1) en série temporelle,
**caractérisé par le fait que** l'appareil d'affichage d'image (4) comprend :
une unité de commande (15) qui commande l'unité d'affichage (11) pour afficher une échelle temporelle (140) indiquant une période d'imagerie de la série d'images, afficher une image spécifique (SP1-5) spécifiant une image souhaitée choisie parmi la série d'images, l'unité de commande (15) étant adaptée pour acquérir des informations de date et d'heure d'imagerie de l'image souhaitée (SP1-5), calculer un temps écoulé depuis la date et l'heure de début d'imagerie jusqu'à ce que l'image souhaitée (SP1-5) soit prise sur la base des informations de date et d'heure d'imagerie et des informations de date et d'heure de début d'imagerie, déterminer une position sur l'échelle temporelle (140) associée à l'image souhaitée (SP1-5), et commander l'unité d'affichage (11) pour afficher une ligne reliant l'image spécifique (SP1-5) et la position sur l'échelle temporelle (140) correspondant à une heure d'imagerie de l'image souhaitée (SP1-5).

2. Appareil d'affichage d'image selon la revendication 1, dans lequel l'unité de commande (15) commande l'unité d'affichage (11) pour afficher, au plus, un nombre prédéterminé d'images souhaitées (SP1-5) spécifiant les images souhaitées sur le même écran et afficher l'index pour chacune des images souhaitées (SP1-5) affichées sur le même écran.

3. Appareil d'affichage d'image selon la revendication 2, comprenant une unité de désignation d'image (10) qui désigne une image souhaitée (SP1-5) d'un objet affiché de l'index à partir des images souhaitées (SP1-5) affichées sur le même écran, l'unité de commande (15) commandant l'unité d'affichage (10) pour afficher l'index pour chacune des images souhaitées (SP1-5) désignées par l'unité de désignation d'image (10).

4. Appareil d'affichage d'image selon la revendication 1, dans lequel l'image souhaitée (SP1-5) est une image mignature correspondant à l'image désignée.

5. Appareil d'affichage d'image selon l'une des revendications 1 à 4, dans lequel le sujet est un candidat soumis à un examen de corps interne, et l'unité de commande (15) détecte une période d'imagerie d'images de corps externe prises par imagerie d'un corps externe du candidat, et commande l'unité d'affichage pour afficher un repère de période d'image de corps externe (142) indiquant une période d'imagerie des images de corps externe de la période d'imagerie de la série d'images.

6. Appareil d'affichage d'image selon l'une des revendications 1 à 4, dans lequel l'unité de commande (15) détecte une période d'imagerie pendant laquelle des images identiques se suivent dans la série d'images, et l'unité de commande commande l'unité d'affichage pour afficher un repère de période d'images identiques (143, 144) indiquant la période d'imagerie des images identiques de la période d'imagerie de la série d'images.

7. Programme d'affichage d'image qui affiche une série d'images prises par imagerie d'un sujet souhaité (1) en série temporelle et affiche une échelle temporelle (140) indiquant une période d'imagerie de la série d'images, et qui amène un ordinateur à exécuter :
une procédure d'extraction d'informations pour extraire des informations correspondant à une heure d'imagerie d'une image souhaitée (101) choisie parmi la série d'images ;
**caractérisé par** l'affichage d'une image spécifique (SP1-5) spécifiant une image souhaitée choisie parmi la série d'images, l'acquisition d'informations de date et d'heure d'imagerie de l'image souhaitée (SP1-5), pour calculer un temps écoulé depuis la date et l'heure de début d'imagerie jusqu'à ce que l'image souhaitée (SP1-5) soit prise sur la base des informations de date et d'heure d'imagerie et des informations de date et d'heure de début d'imagerie, pour déterminer une position sur l'échelle temporelle (140) associée à l'image souhaitée (SP1-5), et l'affichage d'une ligne reliant l'image spécifique (SP1-5) et une position sur l'échelle temporelle (140) correspondant à l'heure d'imagerie de l'image souhaitée (SP1-5).

8. Appareil d'affichage d'image selon l'une quelconque des revendications précédentes, dans lequel :
l'appareil d'affichage comprend en outre une unité d'entrée (10) ; et
les images souhaitées sont choisies lorsqu'un utilisateur réalise l'une parmi une opération de touche et une opération de clic pour sélectionner une image affichée sur une zone d'affichage d'image principale (100) affichée sur l'unité d'affichage (11) à l'aide de l'unité d'entrée (10).

9. Appareil d'affichage d'image selon la revendication 8, dans lequel une pluralité d'images souhaitées (SP1-5) sont choisies par l'utilisateur réalisant l'une parmi l'opération de touche et l'opération de clic.
